# FASCICULE DE BREVET EUROPEEN

(11) **EP 1 510 533 B1**
(45) Date de publication et mention de la délivrance du brevet: **06.12.2006**
(21) Numéro de dépôt: 04104150.0
(22) Date de dépôt: 30.08.2004
(51) Int. Cl.: C08F 293/00

(54) **Copolymères éthyléniques sequences comprenant une sequence vinyllactame, compositions cosmétiques les contenant, et utilisation de ces copolymères en cosmétique**
Blockcopolymere enthaltend ein Vinylllactamblock, kosmetische Zusammensetzung und Verwendung dieser Polymere in der Kosmetik
Block copolymer comprising a vinylactam block, cosmetic composition and use of this polymer in cosmetics

(30) Priorité: 01.09.2003 FR 0350485
(43) Date de publication de la demande: 02.03.2005
(73) Titulaire: L'ORÉAL, 75008 Paris (FR)
(72) Inventeur: Mougin, Nathalie, 75011, PARIS (FR)
(74) Mandataire: Poulin, Gérard

(56) Documents cités:
- DE-A- 10 029 699
- FR-A- 2 327 761
- US-A- 3 739 042
- US-B1- 6 369 165

## Description

### DOMAINE TECHNIQUE

La présente invention a trait à de nouveaux polymères de structure spécifique de type copolymère éthylénique séquencé comprenant une séquence vinyllactame.

La présente invention concerne, en outre, une composition, notamment cosmétique ou pharmaceutique en particulier une composition capillaire, comprenant ledit polymère de structure spécifique.

L'invention a également trait à l'utilisation de ces polymères en cosmétique pour le traitement des matières kératiniques, notamment de la peau, des ongles ou des cheveux.

Précisons que par « séquence vinyllactame », on entend une séquence comprenant un motif lactame, par exemple des dérivés de lactames, en d'autres termes, de manière générale, une séquence susceptible d'être préparée par polymérisation d'un monomère à cycle lactame.

De nombreuses compositions cosmétiques, et en particulier les compositions capillaires, dites compositions de "hair styling", qui se présentent sous la forme d'aérosols ("sprays") de gels, de mousses ou de shampooings contiennent des résines ou polymères.

Il s'agit, en particulier, de polymères acryliques ayant des températures de transition vitreuse (Tg) élevées, tels que ceux décrits dans le document FR-A-2 439 798.

De tels polymères, apportent, notamment en coiffage, un maintien de la chevelure, mais ils présentent l'inconvénient d'une trop grande friabilité, ce qui ne permet pas une bonne tenue dans le temps de la chevelure.

Dans le cas des vernis, les polymères existants ne résistent pas aux chocs.

Pour résoudre les problèmes posés par ces polymères, on utilise, en outre, dans les compositions cosmétiques, des plastifiants, afin d'abaisser la température de transition vitreuse. Mais, alors, les polymères tendent à présenter des effets de « collants » ou, dans le cas du coiffage ne permettent pas de fixation « ultra-forte ».

En outre, lorsque les cheveux sont coiffés, nombreux sont les polymères de coiffage ("hair styling") existant qui forment des particules blanches, ce qui n'est pas acceptable, en particulier lorsque les cheveux sont bruns et/ou épais. D'autres inconvénients présentés par les polymères utilisés à l'heure actuelle sont leur incompatibilité avec les propulseurs pour aérosols existant.

On connaît aussi, par ailleurs, dans le domaine de la cosmétique des polymères à base de vinyllactames par exemple de vinylpyrrolidone, et en particulier d'homopolyvinylpyrrolidone. Le principal défaut de ces polymères est leur forte hygroscopie, qui conduit à leur donner en présence de l'humidité ambiante, un fort caractère collant.

Ainsi, le document EP-A-1 002 811 de BASF décrit des polymères greffés solubles ou dispersibles dans l'eau obtenus par polymérisation radicalaire de monomères essentiellement acryliques et d'un prépolymère polymérisable à base de vinyllactame par exemple de vinylpyrrolidone ou de vinylcaprolactame.

Ces polymères sont utilisés notamment dans des compositions capillaires.

Le document US-A-6 193 961 de ISP décrit un terpolymère homogène de N-vinyllactame de préférence de N-vinylpyrrolidone ou de N-vinyl-caprolactame, d'acrylate de diméthylaminoalkyle ou de diméthylaminoalkylacrylamide et d'un monomère polysiloxane.

Ces terpolymères sont utilisés dans des compositions cosmétiques et de soins par exemple des compositions cosmétiques telles que des gels coiffants ou des mousses.

Dans ce document sont cités de nombreux autres brevets mentionnant l'utilisation de polymères à base de vinyllactame dans les compositions de soins pour la peau et les cheveux, tels que les documents US-A-3 914 403, US-A-3 954 960, US-A-4 039 734, US-A-4 057 533, US-A-4 210 161, US-A-4 223 009, US-A-4 586 518, US-A-4 764 363, US-A-4 834 968, US-4 842 850, US-A-4 902 499, US-A-4 906 459, US-A-4 923 694, US-4 963 348, US-A-5 011 895, US-A-5 015 708, US-A-5 126 124, US-A-5 158 762, US-A-5 275 809, US-A-5 502 136, WO-A-91/15186, WO-A-91/15185, EP-A2-412704, EP-A1-0412707 et JP-A-57126409.

Dans ce même document, on indique également que de nombreux brevets décrivent l'utilisation, en particulier, d'une N-vinyllactame dans le domaine de la cosmétique et des produits pharmaceutiques, en particulier dans les aérosols pour les cheveux. Ces brevets sont les documents US-A-3 910 862, US-A-4 923 94, US-A-5 045 617, US-A-5 321 110, US-A-5 492 988 et US-A-5 637 296.

Ainsi, le document US-A-3 954 960 a trait à des compositions cosmétiques et capillaires qui contiennent en tant que résine filmogène un copolymère quaternisé à base de vinylpyrrolidone et de monomère vinylique copolymérisable à savoir un méth(acrylate) de dialkylaminoalkyle.

Le brevet US-A-3 914 403, quant à lui, est relatif à des compositions capillaires qui contiennent un mélange de résines filmogènes : un homo ou copolymère de N-vinylpyrrolidone en mélange avec un copolymère quaternisé à base de vinylpyrrolidone et de monomère vinylique copolymérisable avec celle-ci à savoir un méth(acrylate) de dialkylaminoalkyle.

Le document US-A-5 502 136 concerne un procédé de préparation de copolymères de vinylpyrrolidone et d'acétate de vinyle par polymérisation radicalaire.

Le document WO-A-00/68282 a trait à des terpolymères à base de vinylpyrrolidone (VP), de diméthylaminopropyl méthacrylamide (DMAPMA), et du dérivé quaternisé par une chaîne alkyle en C₈ à C₂₄ du DMAPMA, et aux compositions capillaires et cosmétiques les comprenant.

On note que dans ces copolymères des chaînes grasses sont insérées afin de diminuer le collant et d'augmenter la résistance à l'humidité, mais la gamme des propriétés des polymères obtenus se trouve ainsi limitée. Par ailleurs, aucune valeur du collant « tack » de ces copolymères n'est mentionnée.

Le document de Matyjasezwski et al., Prepr. 38(1), 1997, p. 695 décrit des copolymères à squelette N-vinylpyrrolidone et greffons PDMS pour des hydrogels.

Le brevet WO 97/18247 du même K. Matyjaszewski décrit, à la page 103, un exemple d'homopolymère de vinylpyrrolidone.

Le document FR-A-2 327 761 est relatif à des compositions cosmétiques comprenant un polymère résultant de la polymérisation, en présence de cérium, d'un monomère insaturé sur un polyvinylpyrrolidone diol. Un polymère PVP-poly(méthacrylate de lauryle) est mentionné. L'utilisation d'un copolymère vinylpyrrolidone/acrylate de glucosamine est également décrite. Les polymères de ce document sont des polymères très particuliers, généralement à structure branchée.

Ces polymères apportent brillance et tenue dans le temps à la coiffure.

Mais ils confèrent aussi une certaine rigidité à la coiffure causant un aspect non-naturel. Ils présentent en outre l'inconvénient d'une forte hygroscopie de par la présence des motifs hydroxy n'ayant pas réagi, le long des chaînes de polymères.

Il existe donc un besoin pour un polymère, qui, lorsqu'il est inclus dans une composition, en particulier une composition cosmétique, fasse en sorte que cette composition ne présente pas les inconvénients, défauts, limitations et désavantages des compositions de l'art antérieur.

Il existe, en particulier, un besoin pour un polymère et une composition le contenant, qui présente une combinaison optimale des propriétés de rigidité et de « collant ».

Ainsi, une composition capillaire comportant le polymère doit permettre d'obtenir plus de tenue, tout en conservant un effet naturel. Le polymère doit notamment, dans de telles compositions, montrer de bonnes propriétés de coiffage « styling » et, lors du démêlage, ne pas poudrer c'est à dire ne pas former des résidus visibles " flaking ". Par ailleurs, le polymère doit être compatible avec les gaz propulseurs aérosols.

Dans le cas du maquillage des ongles l'obtention d'un film brillant est souhaité, ce film devant, en plus, résister aux agressions mécaniques. Le polymère contenu dans la formule doit donc être capable de résister de manière excellente à l'abrasion mécanique.

Dans le cas d'un traitement de la peau, le maquillage mis en oeuvre et qui inclut le polymère, doit adhérer à la peau, tout en étant confortable (ne pas provoquer des « tiraillements»).

Dans tous les cas et quelle que soit la composition dans laquelle se trouve utilisé le polymère, il est nécessaire que ce dernier donne un produit non collant au toucher, en particulier dans des conditions d'humidité élevée.

En d'autres termes, il existe un besoin pour un polymère filmogène possédant les propriétés optimales de rigidité afin d'accéder à des produits tels que laques ou vernis à ongles à effet naturel, ne présentant pas ou peu de collant ou « tack ».

Le but de la présente invention est de fournir un polymère qui réponde, entre autres, aux besoins, critères et exigences cités plus haut et qui résolve les problèmes des polymères de l'art antérieur.

Ce but et d'autres encore sont atteints, conformément à la présente invention, par un copolymère éthylénique, séquencé, linéaire comprenant :
- au moins une séquence A susceptible d'être préparée à partir de monomères, comprenant de 52 à 100% en poids d'un monomère éthylénique à cycle lactame répondant à la formule (I) suivante : 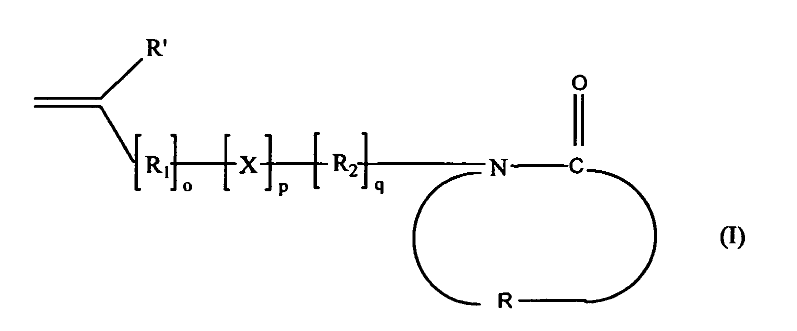 dans laquelle :
   - R représente un groupe -(CH₂)ₙ-, où un ou plusieurs des atomes de carbone sont éventuellement remplacés par un atome d'azote ou un atome d'oxygène, où n est un nombre entier de 3 à 12, et où un ou plusieurs des atomes de carbone sont éventuellement substitués par un ou plusieurs groupes alkyle en C₁ à C₆ ;
   - R' représente H ou un groupe méthyle ;
   - R₁ et R₂, qui peuvent être identiques ou différents représentent un groupe alkylène ou aralkylène, linéaire, ramifié, ou cyclique, de 1 à 22 C, dans lequel un ou plusieurs des atomes de carbone sont éventuellement remplacés par un atome d'oxygène ou d'azote ;
   - X est choisi parmi -OCO-, -NHCO-, -COO-, -O- ;
   - o, p, q, représentent chacun indépendamment les uns des autres 0 ou 1 ; ladite séquence A représentant de 10 à 95% en poids du copolymère ;
- et au moins une séquence B susceptible d'être préparée à partir de monomères ne comportant pas de monomère éthylénique à cycle lactame de formule (I) ou comportant une proportion minoritaire de celui-ci, ladite séquence B présentant une température de transition vitreuse Tg comprise entre -55°C et +55°C. Ladite séquence B représentant de 5 à 90% en poids du copolymère.

La teneur en monomère éthylénique à cycle lactame de 52 à 100% en poids est donnée par rapport au poids total des monomères à partir desquels est susceptible d'être préparée la séquence A.

En d'autres termes, la teneur en motifs issus d'un monomère éthylénique à cycle lactame de la séquence A est de 52 à 100% en poids ;
cette teneur de 52 à 100% en poids est donnée par rapport au poids total de la séquence A.

De préférence, dans la formule (I), o = 0, p = 1, q = 1,R₂ représente -CH₂CH₂-, X représente COO ou CONH, et R est (CH₂)₃ ou (CH₂)₅ ou (CH₂CH₂NH).

Le monomère de formule (I) sera donc de préférence un méthacrylate ou acrylate de pyrrilidono éthyle, ou un acrylate, ou un méthacrylate d'uréido éthyle.

De préférence encore, la séquence A est susceptible d'être préparée à partir de monomères, comprenant de 52 à 100% en poids d'un vinyllactame, répondant à la formule (II) suivante : 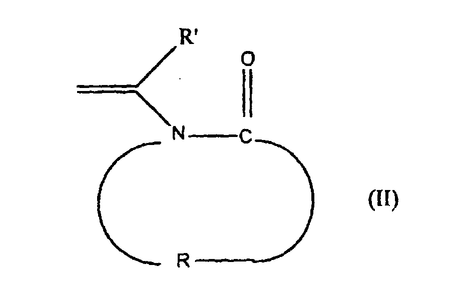 dans laquelle R et R' ont la signification déjà donnée plus haut.

De préférence, dans les formules (I) et (II) ci-dessus, R représente -(CH₂)n- avec n = 3 à 5, ou bien R est -CH₂-CH₂-NH-.

L'invention a également pour objet les compositions cosmétiques comprenant lesdits copolymères éthyléniques, séquences, linéaires. Les copolymères inclus dans ces compositions étant toutefois tels que ladite séquence A et ladite séquence B représentent chacune respectivement 1 à 99% en poids du poids total du copolymère.

Lorsqu'ils sont incorporés dans de telles compositions, les copolymères présentant la structure spécifique, selon l'invention, et dans lesquels ladite séquence A et ladite séquence B représentent alors chacune respectivement de 1 à 99% en poids du poids total du copolymère, permettent d'obtenir des propriétés, ou plutôt une combinaison de propriétés extrêmement intéressantes, qu'il n'était pas possible d'obtenir avec les polymères de l'art antérieur.

De manière générale, les copolymères selon l'invention présentent, du fait de leur structure particulière une hygroscopie réduite par rapport aux copolymères de l'art antérieur présentant des motifs vinyllactames.

Les copolymères de l'invention ont une combinaison optimale de rigidité et de caractère non collant et ils conduisent ainsi à des compositions ou systèmes ayant notamment des résistances mécaniques, des résistances à l'usure et des tenues dans le temps améliorées, et une fragilité réduite, tout en n'étant pas collants.

Les copolymères selon l'invention peuvent être définis comme des polymères filmogènes et ne présentant pas de collant ou « tack ».

Ainsi, lorsque les copolymères, selon l'invention, sont utilisés dans des compositions pour le traitement de la chevelure, telles que des laques, ils apportent plus de tenue dans le temps. Ils sont moins fragiles qu'une laque classique et simultanément ils ne sont pas collants. Le phénomène de poudrage sur les cheveux observé, lors du démêlage, avec les compositions de l'art antérieur est évité.

Dans le cas des vernis à ongles, la formule comprenant le copolymère selon l'invention, a une résistance à l'usure supérieure et ne colle pas, tout en adhérant sur l'ongle. De ce fait, la perte de brillance, c'est-à-dire la matification du film par le marquage mécanique ou par le marquage par les poussières qui se produit avec des films collants de l'art antérieur ne se produit pas avec les vernis et films comprenant le copolymère selon l'invention. En effet, l'absence de collant, « tack », de ces derniers fait qu'ils ne sont pas marqués mécaniquement et qu'ils ne retiennent pas les poussières et qu'ils ne subissent donc pas de modification ou perte de brillance.

Les vernis, comprenant le copolymère selon l'invention, donnent des films qui ne s'écaillent pas, grâce notamment à la présence dans le copolymère selon l'invention d'une séquence B présentant une Tg très particulière, se situant dans une plage précise, qui empêche l'écaillage des films, mais simultanément et de manière étonnante ne conduit pas à un film collant.

Dans les produits de maquillage, comme les rouges à lèvres ou les fonds de teint, le maquillage présente une bonne tenue sur les lèvres ou la peau, sans laisser de sensation de collant.

L'invention concerne également un procédé cosmétique de maquillage ou de soin des matières kératiniques comprenant l'application sur les matières kératiniques d'une composition selon l'invention.

L'invention concerne donc en outre l'utilisation des copolymères selon l'invention, dans lesquels ladite séquence A et ladite séquence B représentent alors chacune respectivement de 1 à 99% en poids du poids total du copolymère, pour améliorer la tenue de la coiffure, sans collant, d'une laque pour cheveux ; l'utilisation des copolymères selon l'invention, dans lesquels ladite séquence A et ladite séquence B représentent alors chacune respectivement de 1 à 99% en poids du poids total du copolymère, pour améliorer l'adhérence et la résistance à l'usure, sans collant, d'un vernis à ongles et enfin l'utilisation des copolymères selon l'invention, et dans lesquels ladite séquence A et ladite séquence B représentent alors chacune respectivement de 1 à 99% en poids du poids total du copolymère,dans une composition cosmétique telle qu'une composition de maquillage pour masquer les rides, qui donne à la peau un aspect lissé, sans tiraillement.

Les copolymères, selon l'invention, apportent donc une solution aux problèmes posés par les polymères de l'art antérieur.

Les propriétés avantageuses inattendues des copolymères spécifiques de l'invention, qui sont fondamentalement des copolymères linéaires, proviennent notamment, voire essentiellement, de la nature spécifique des séquences qui les constituent.

De manière étonnante, les copolymères selon l'invention, grâce, semble-t-il, à la présence d'une séquence (séquence B) exempte de monomère éthylénique à cycle lactame tel qu'un vinyllactame, ou dans laquelle le monomère éthylénique à cycle lactame tel qu'un vinyllactame est en minorité, ont une hygroscopie réduite par rapport aux polymères à base de vinyllactame de l'art antérieur et notamment par rapport aux homopolymères de vinyllactame, tels que l'homopolyvinylpyrrolidone.

En outre, il a été mis en évidence, de manière surprenante, selon l'invention, que la présence dans le polymère selon l'invention des séquences spécifiques B présentant des Tg très spécifiques permettrait de contrôler la friabilité du copolymère et d'obtenir, de manière surprenante, des copolymères qui ne sont pas fragiles, friables, qui donnent des films flexibles et qui ne poudrent pas, tout en n'étant pas collants ; on surmonte ainsi l'un des inconvénients essentiels des polymères à base de polyvinyllactame de l'art antérieur qui sont relativement friables, tout en étant collants.

Rien ne laissait supposer dans l'art antérieur qu'en mettant en oeuvre un copolymère spécifiquement linéaire, en imposant qu'au moins une séquence (A) de ce copolymère comprenne de 52 à 100% en poids d'un monomère éthylénique à cycle lactame tel qu'un vinyllactame, qu'au moins une autre séquence (B) ait une structure différente de la séquence (A) et soit exempte de ou comprenne de 52 à 100% en poids d'un monomère éthylénique à cycle lactame tel qu'un N-vinyllactame, et enfin que la séquence B ait une température de transition vitreuse spécifique comprise entre -55°C et +55°C, on pourrait parvenir, selon l'invention, à obtenir une combinaison de propriétés excellentes pour ce copolymère. La structure spécifique du copolymère de l'invention conduit à une optimisation de ses propriétés, conduisant à un parfait équilibre entre les propriétés de résistance mécanique, et de non-collant.

Sans vouloir être lié par aucune théorie, les propriétés avantages du copolymère selon l'invention proviendraient du fait que la nature des séquences est spécifiquement choisie de façon à favoriser la séparation des phases entre celles-ci et donc, entre autres, à contrôler de manière optimale la rigidité et l'hygroscopie et donc le collant du copolymère. Par ailleurs, le fait que le polymère soit linéaire implique une synthèse beaucoup plus facile et contrôlée, ce qui permet de prévoir de façon précise la structure des polymères obtenus, et ainsi d'optimiser les propriétés finales des polymères.

De manière plus précise, les copolymères selon l'invention sont des copolymères séquences ou blocs. On entend généralement par ces termes que les copolymères sont constitués de séquences ou blocs accrochés les uns aux autres de façon covalente.

En outre, deux séquences successives sont de natures différentes. Par contre, deux séquences A ou B non successives peuvent être de même nature. Chaque séquence A ou B peut être constituée d'un homopolymère ou d'un copolymère, celui-ci pouvant à son tour être statistique ou alterné ou à gradient.

Le polymère peut comporter en outre une autre séquence C différente des séquences A et B et éventuellement encore d'autres séquences par exemple une séquence D différente de A, B et C.

Le polymère selon l'invention pourra donc être choisis parmi les copolymères biséquencés de type AB, les copolymères triséquencés de type ABA, BAB, ABC, ACB, avec C différent de A ou B.

Le polymère selon l'invention peut aussi être choisi parmi les copolymères multiséquencés ayant plus de trois séquences différentes ou non : (AB)ₙ, (ABA)ₙ, (BAB)n, (ABC)n, (ABC)n avec C différent de A ou B, ou les copolymères multiséquencés ayant plus de trois séquences différentes ABCD.

De manière générale, comme on l'a déjà indiqué, la nature des séquences est choisie de manière à favoriser la séparation des phases entre les séquences, puisque celle ci prédétermine les propriétés.

La nature des séquences et leur nombre sont choisis par l'homme du métier en fonction des propriétés recherchées dans les limites des conditions spécifiées ci-dessus pour les séquences A et B.

Les copolymères de l'invention sont définis comme étant des copolymères éthyléniques. Cela signifie que les monomères dont sont issus les séquences ou blocs constituant le copolymère sont des monomères à double liaison insaturée carbone-carbone de type éthylénique.

En outre, spécifiquement, le copolymère, selon l'invention, est un copolymère linéaire. Cela signifie que l'invention n'entend pas couvrir les copolymères ayant une structure non linéaire, par exemple ramifiée, en étoile, greffée, ou autre. Le caractère linéaire des copolymères de l'invention est important pour communiquer aux compositions le contenant, les propriétés avantageuses décrites plus haut.

Avantageusement, le copolymère est un polymère filmogène, c'est-à-dire qu'il est apte à lui seul, ou en présence d'agent auxiliaire de filmification, à la température allant de 20°C à 30°C, à former un film continu (vu à l'oeil nu) et adhèrent sur un support kératinique.

Selon l'invention, le copolymère comprend au moins une séquence A susceptible d'être préparée à partir de monomères comprenant de 52 à 100% en poids d'un monomère éthylénique à cycle lactame, par exemple d'un vinyllactame, de formule (I), et de préférence de formule (II).

La teneur en monomère éthylénique à cycle lactame de 52 à 100% en poids est donnée par rapport au poids total des monomères à partir desquels est susceptible d'être préparée la séquence A.

En d'autres termes, la teneur en motifs issus d'un monomère éthylénique à cycle lactame de la séquence A est de 52 à 100% en poids.

Cette teneur de 52 à 100% en poids est donnée par rapport au poids total de la séquence A.

Généralement, le pourcentage de monomère éthylénique à cycle lactame, tel qu'un vinyllactame, de formule (I) ou (II), dans les monomères à partir desquels la séquence A est susceptible d'être préparée, est de préférence compris entre 55 à 95 % en poids, par exemple il est de 90 % en poids.

En d'autres termes, la teneur en motifs issus d'un monomère éthylénique à cycle lactame de la séquence A est de préférence comprise entre 55 et 95% en poids, par exemple elle est de 90% en poids ;
ces teneurs en poids sont données par rapport au poids total de la séquence A.

La séquence A comprenant de 52 à 100% en poids de vinyllactame peut avoir une température de transition vitreuse Tg quelconque, mais elle a généralement une température de transition vitreuse globale de la séquence « haute ». Par haute, on entend généralement que cette Tg peut être comprise entre 0 et 250°C, de préférence entre 0 et 220°C et de préférence encore entre 5 et 200°C.

Par exemple, la température de transition vitreuse Tg d'une séquence A constituée d'homopolyvinyllactame est généralement très supérieure à 100°C à une HR de 0 %.

La séquence A peut être hydrophile ou hydrophobe. Une séquence hydrophile peut être définie comme une séquence hydrosoluble ou hydrodispersible. De préférence, la séquence A est une séquence hydrophile. Le terme hydrophile est défini plus bas.

Avantageusement, la séquence A est un homopolymère du monomère éthylénique à cycle lactame, tel que le N-vinyllactame, de formule (I) ou de formule (II).

Le monomère de N-vinyllactame peut être choisi parmi les N-vinyllactames et les dérivés de ceux-ci qui peuvent avoir, par exemple, un ou plusieurs substituants alkyle en C₁ à C₆, tels que méthyle, éthyle n-propyle, isopropyle, n-butyle, sec-butyle.

Avantageusement, ledit N-vinyllactame de formule (I) est le pyrrilidinoéthylacrylate ou le pyrrilidinoéthylméthacrylate.

Avantageusement, le N-vinyllactame de formule (II) est le N-vinylpyrrolidone (n=3), le N-vinylpipéridinone (valérolactame) (n=4), le N-vinylcaprolactame (n=5), le N-vinylimidazolidinone dans laquelle R est le groupe -CH₂-CH₂-NH-, le N-vinyl-5-méthyl-2-pyrrolidone, le N-vinyl-5-éthyl-2-pyrrolidone, le N-vinyl-6-méthyl-2-pipéridone le N-vinyl-6-éthyl-2-pipéridone, le N-vinyl-7-méthyl-2-caprolactame, ou le N-vinyl-7-éthyl-2-caprolactame.

De préférence, en relation avec le choix du N-vinyllactame, lorsque la séquence A est un homopolymère, c'est un homopolymère de N-vinylpyrrolidone ou de N-vinylcaprolactame.

Lorsque la séquence A, comprenant de 52 à 100% en poids d'un monomère éthylénique à cycle lactame, tel que le N-vinyllactame, est un copolymère, les monomères, à partir desquels elle est susceptible d'être préparée, comprennent, outre le monomère éthylénique à cycle lactame, tel que le vinyllactame de formule (I), un ou plusieurs autres monomères.

De préférence, indépendamment du choix du N-vinyllactame, la séquence A est un copolymère car cela permet de moduler les propriétés, en particulier de moduler les propriétés de collant.

Ce ou ces monomères de la séquence A, autres que le monomère éthylénique à cycle lactame, tel que le vinyllactame, sont des monomères éthyléniques copolymérisables avec le monomère dérivé lactame quelque soit leur coefficient de réactivité. Ce ou ces monomères de la séquence A, autres que le monomère éthylénique à cycle lactame, tel que le vinyllactame, sont choisis, de préférence, parmi les monomères dont la Tg de l'homopolymère correspondant est inférieure ou égale à 50°C, de préférence encore inférieure ou égale à 20°C, mieux inférieure ou égale à 0°C ; de préférence, les monomères ont, en outre, une Tg de l'homopolymère correspondant qui est supérieure ou égale à -150°C.

Dans la séquence A, le ou les monomères, autres que le monomère éthylénique à cycle lactame comme un vinyllactame, peuvent être hydrophiles ou non hydrophiles.

En particulier, dans la séquence A, un ou plusieurs parmi les monomères autres que le monomère éthylénique à cycle lactame, comme un vinyllactame, sont choisis parmi les monomères hydrophiles.

Les monomères hydrophiles sont choisis, par exemple, parmi les monomères hydrophiles décrits plus loin. Des exemples de monomères hydrophiles incluent les monomères cationiques, les monomères anioniques et les monomères non ioniques décrits plus bas.

Un ou plusieurs monomères autres que le monomère éthylénique à cycle lactame, comme un vinyllactame, peuvent être choisis parmi les monomères non hydrophiles. Ces monomères non hydrophiles sont choisis parmi la liste des monomères pouvant constituer la séquence B, en respectant les critères de Tg et d'hydrophilie souhaités.

Le ou les monomère (s) autre (s) que le monomère éthylénique à cycle lactame, comme un vinyllactame, dont la Tg de l'homopolymère correspondant est inférieure ou égale à 50°C, préférés sont choisis parmi : l'acrylate de t-butyle (Tg=50°C) , l'acétate de vinyle (Tg=23°C) , le méthacrylate de butyle (Tg=20°C), l'acrylate de cyclohexyle (19°C) , l'acrylate d'hydroxyéthyle (15°C), l'acrylate de méthyle (Tg=10°C),le méthacrylate d'éthoxyéthyle (Tg=0°C), le méthacrylate de n-hexyle ( -5°C),l'acrylate d'éthyle (Tg=-24°C), l'acrylate d'isobutyle (-24°C), le vinylméthyle ether (-34°C), l'acrylate de méthoxyéthyle (-33°C), l'acrylate de n-butyle (Tg=-54°C),l'acrylate d'éthylhexyle (Tg=-50°C), le méthacrylate de POE (n=8 à 10) (Tg=-55°C), et le méthacrylate d'isobornyle ;le butylacrylamide, le N-isopropylacrylamide, le N,N-diméthylacrylamide et le N,N-dibutylacrylamide, l'acrylate d'hexyle, l'acrylate d'octyle, l'acrylate de lauryle, l'acrylate d'isooctyle, le méthacrylate d'éthylhéxyle, le méthacrylate d'octyle,le méthacrylate de lauryle, le néononanoate de vinyle, et le néododécanoate de vinyle ; le N-octylacrylamide.

La séquence A, comprenant de 52 à 100% en poids en monomère éthylénique à cycle lactame, représente de 1 à 99 % en poids du copolymère, de préférence de 10 à 95 %, de préférence encore de 20 à 90 % en poids du poids total du copolymère final. Lorsque le copolymère est inclus dans une composition cosmétique selon l'invention, ou utilisé selon l'invention, alors la séquence A représente de 1 à 99% en poids du copolymère.

La ou les séquences B autres que la séquence A est(sont) susceptible(s) d'être préparée(s) à partir d'un ou de plusieurs monomères éthyléniques choisis généralement parmi : les monomères allyliques, les acrylates, les méthacrylates, les acrylamides, les méthacrylamides, les monomères vinyliques, et leurs mélanges, et éventuellement des monomères éthyléniques à cycle lactame, par exemple des monomères de vinyllactame, de formule (I) ou (II), la proportion en poids desdits monomères (I) ou (II) étant minoritaire, à savoir inférieure à 50 % en poids, de préférence inférieure ou égale à 40 % et, de préférence encore inférieure ou égale à 30% en poids.

Avantageusement, la masse moléculaire en nombre de chaque bloc ou séquence, qu'il s'agisse de la séquence A ou de la séquence B est comprise entre 2000 et 1 000 000, de préférence encore comprise entre 2000 et 800 000, et mieux comprise entre 2000 et 500 000.

De manière plus précise, selon l'invention, le copolymère comprend au moins une séquence ou bloc qui a une température de transition vitreuse spécifique se trouvant dans l'intervalle allant de -55°C à +55°C.

La température de transition vitreuse Tg étant un paramètre essentiel pour définir les séquences du copolymère de l'invention, notamment la séquence B du copolymère de l'invention et, par voie de conséquence, le copolymère de l'invention, il est important d'indiquer que les températures de transition vitreuse des séquences des copolymères utilisés dans la présente invention sont mesurées par analyse enthalpique différentielle (DSC, « Differential Scanning Calorimetry », en anglais) pour le polymère sec, à une vitesse de chauffe de 10°C/minute.

Chaque séquence du copolymère, selon l'invention, est issue d'un type de monomère ou de plusieurs types de monomères différents.

Cela signifie que chaque séquence peut être constituée d'un homopolymère ou d'un copolymère ; ce copolymère constituant la séquence pouvant être à son tour statistique ou alterné ou à gradient. Dans le cas de la séquence A, il s'agit bien sûr d'un homopolymère de vinyllactame ou d'un copolymère comprenant une proportion d'au moins 52% de monomère éthylénique à cycle lactame tel qu'un vinyllactame.

La masse moléculaire en nombre du copolymère global (par exemple A-b-B) est généralement comprise entre 4 000 et 1 000 000, de préférence entre 4 000 et 800 000, de préférence encore entre 4 000 et 500 000.

Avantageusement, la proportion de la séquence B de Tg globale comprise entre -55°C et +55°C est de 1 % à 99% en poids du copolymère, de préférence de 5 à 90 % et, de préférence encore de 10 à 80 % en poids total du polymère final. Lorsque le copolymère est inclus dans une composition cosmétique selon l'invention, ou utilisé selon l'invention, alors la séquence B représente de 1 à 99% en poids du copolymère.

Avantageusement, la séquence B dont la Tg peut varier entre -55°C et 55°C a une température Tg pouvant varier dans l'intervalle de -50°C et +50°C, de préférence de -50°C à +45°C.

De préférence, le ou les monomères constituant la séquence B satisfaisant la condition de Tg ci-dessus ont une longueur de chaîne carbonée inférieure ou égale à 10 atomes de carbone, en effet cela aura tendance à diminuer le collant de la séquence considérée, ou alors si les chaînes carbonées ont une longueur de chaîne carbonée supérieure ou égale à 12 atomes de carbone, la proportion de ces monomères dans la séquence B est faible, à savoir généralement inférieure à 50 % en poids. Par chaîne carbonée, on entend généralement la chaîne portée par le motif éthylénique et donc pendante sur le squelette.

La séquence B, dont la température de transition vitreuse globale (présentée par la séquence prise dans son ensemble) est comprise entre -55°C et +55°C, est un homopolymère ou copolymère, issu de un ou plusieurs monomères qui sont tels que les homopolymères préparés à partir de ces monomères peuvent présenter des températures de transition vitreuse variant de -200 à +250°C.

L'homme du métier choisit ces monomères et leurs proportions de façon à ce que la Tg globale de la séquence soit dans la plage voulue.

Une séquence B de Tg adéquate comprise entre -55 et 55°C, pourra ainsi être formée d'un copolymère constitué à partir d'un premier monomère dont la Tg de l'homopolymère correspondant est dans la plage de +20°C à +250°C, tel que le méthacrylate de méthyle (MMA) ou le styrène, et d'un second monomère dont la Tg de l'homopolymère correspondant est dans la plage de -200°C à +20°C .

Par exemple, on pourra combiner dans le copolymère, formant la séquence un monomère de Tg (de l'homopolymère correspondant) égale à 100°C, par exemple le méthacrylate de méthyle, à raison de 35 % en poids du poids total de monomères, et un monomère de Tg égale à -50°C, par exemple l'acrylate de 2-éthylhexyle, à raison de 65 % en poids, et la séquence résultante aura une Tg de -14°C., calculée en première approximation par la loi de Fox : 1/Tg = Somme (% Wa/Tga) avec Wa= % en poids du monomère a et Tga = température de transition vitreuse de l'homopolymère constitué à partir du monomère a.

La séquence B dont la température de transition vitreuse peut varier de -55°C à +55°C, peut être également un homopolymère, constitué à partir d'un seul type de monomère dont la Tg de l'homopolymère correspondant est bien évidemment dans la plage allant de -55°C à +55°C, de préférence de -50 à +50°C, et de préférence encore de -50 à +45°C. Il est à noter cependant qu'il existe peu de monomères ayant une Tg de l'homopolymère correspondant dans cette plage : la plupart sont mentionnés plus bas.

Le ou les monomères à partir desquels la séquence ou les séquences B est(sont) susceptible(s) d'être préparée(s) peuvent donc, par exemple, être choisi(s) parmi les monomères décrits ci-après.
- Les hydrocarbures éthyléniques de 2 à 10 C, tels que l'éthylène, l'isoprène, ou le butadiène ;
- les acrylates de formule CH₂ = CHCOOR₃ ;
- les méthacrylates de formule : 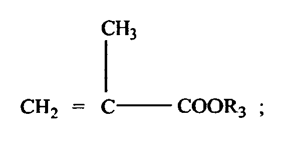 dans lesquelles R₃ représente :
   - un groupe alkyle linéaire, ou ramifié, de 1 à 18 atomes de carbone, dans lequel se trouve(nt) éventuellement intercalé(s) un ou plusieurs hétéroatomes choisis parmi 0, N, S et P,
      ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅) , où R₄ et R₅ identiques ou différents représentent un groupe alkyle en C₁ à C₆ ou un groupe phényle ;
      des exemples de ces groupes alkyle sont méthyle, éthyle, propyle, butyle, isobutyle, tertiobutyle, et ethylhexyle, octyle, lauryle, stéaryle,
      des exemples de ces groupes dérivés d'alkyle (c'est-à-dire alkyle substitués et/ou interrompus) sont les groupes hydroxyalkyle en C₁₋₄ tels que 2-hydroxyéthyle et 2-hydroxypropyle, et les groupes alcoxy (C₁₋₄) alkyle (C₁₋₄) tel que méthoxyéthyle, éthoxyéthyle et méthoxypropyle,
   - un groupe cycloalkyle en C₃ à C₁₂, tel que le groupe isobornyle,
   - un groupe aryle en C₃ à C₂₀ tel que le groupe phényle,
   - un groupe aralkyle en C₄ à C₃₀ (groupe alkyle en C₁ à C₈) tel que 2- phényl-éthyle ou benzyle,
   - un groupe hétérocyclique de 4 à 12 chaînons contenant un ou plusieurs hétéroatomes choisis parmi O, N, et S, le cycle étant aromatique ou non,
   - un groupe hétérocyclylalkyle (alkyle de 1 à 4 C), tel que furfurylméthyle ou tétrahydrofurfurylméthyle,
   lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle, les atomes d'halogène (Cl, Br, I et F), et les groupes Si (R₄R₅) , où R₄ et R₅ identiques ou différents représentent un groupe alkyle en C₁ à C₆, ou un groupe phényle,
   des exemples de groupes R₃ sont les groupes méthyle, éthyle, propyle , isobutyle, n-butyle, tertiobutyle isobutyle, héxyle, éthylhéxyle, octyle, lauryle, isooctyle, isodécyle, dodécyle, cyclohexyle, t-butylcyclohexyle, t-butylbenzyle, d'isobornyle, phényle, furfuryléméthyle, tétrahydrofurfurylmethyle, 2-hydroxyéthyle, 2-hydroxypropyle, 2-hydroxybutyle, méthoxyéthyle, éthoxyéthyle, méthoxyéthyle, méthoxypropyle, éthyl-2-perfluorohexyle,
   - un autre exemple de groupe R₃ sont les groupes R₃ = - (OC₂H₄ ) ₘ-OR" , avec m = 5 à 150 et , R" = H ou alkyle de C₁ à C₃₀, par exemple -POE- méthoxy ; -POE-béhényle ;
      - Les (méth)acrylamides de formule : 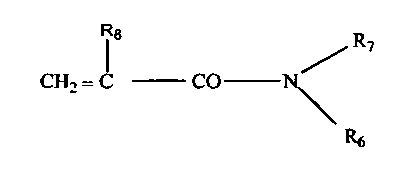 où
         R₈ désigne H ou méthyle ;
         et R₇ et R₆ identiques ou différents représentent chacun un atome d'hydrogène ou un groupe alkyle de 1 à 18 atomes de carbone linéaire ou ramifié, dans lequel se trouve(nt), éventuellement intercalé(s) un ou plusieurs hétéroatomes choisis parmi 0, N, S et P, ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅) , où R₄ et R₅ identiques ou différents, représentent un groupe alkyle en C₁ à C₆, ou un groupe phényle,
         des exemples de ces groupes sont les groupes méthyle, éthyle, n-butyle, t-butyle, isopropyle, isohexyle, isooctyle, isononyle, et hydroxyalkyle en C₁₋₄ tels que 2-hydroxypropyle,
   - un groupe cycloalkyle en C₃ à C₁₂, tel que le groupe isobornyle,
   - un groupe aryle en C₃ à C₂₀ tel que phényle,
   - un groupe aralkyle en C₄ à C₃₀ (groupe alkyle en C₁ à C₈) tel que 2- phényl éthyle ou benzyle,
   - un groupe hétérocyclique de 4 à 12 chaînons contenant un ou plusieurs hétéroatomes choisis parmi O, N, et S, le cycle étant aromatique ou non,
   - un groupe hétérocyclylalkyle (alkyle de 1 à 4 C), tel que furfurylméthyle ou tétrahydrofurfurylméthyle,
   lesdits groupes cycloalkyle, aryle, aralkyle, hétérocyclique ou hétérocyclylalkyle pouvant être éventuellement substitué(s) par un ou plusieurs substituants choisi(s) parmi les groupes hydroxyle, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si (R₄R₅) , où R₄ et R₅ identiques ou différents représentent un groupe alkyle en C₁ à C₆, ou un groupe phényle.

Des exemples de monomères (méth)acrylamide sont le(méth)acrylamide, le N-éthyl(méth)acrylamide, le N-butylacrylamide, le N-t-butylacrylamide, le N-isopropylacrylamide, le N,N-diméthyl(méth)acrylamide, le N,N-dibutylacrylamide, le N-octylacrylamide, le N-dodécylcacrylamide, l'undécylacrylamide, et le N(2-hydroxypropylméthacrylamide).
- Les composés allyliques de formule :

   CH₂ = CH-CH₂-R₉ ou CH₂ = C (CH₃) -CH₂-R₉ ;
- Les composés vinyliques de formule :

   CH₂ = CH-R₉,

   où R₉ est un groupe
   - hydroxyle,
   - Cl,
   - NH₂,
   - OR₁₀, où R₁₀ représente un groupe phényle ou un groupe alkyle en C₁ à C₁₂ (le monomère est un éther de vinyle ou d'allyle),
   - Acétamide : NHCOCH₃,
   - OCOR₁₁, où R₁₁ représente :
      - un groupe alkyle de 2 à 12 C linéaire ou ramifié (le monomère est un ester de vinyle ou d'allyle) ;
      - un groupe cycloalkyle en C₃ à C₁₂ tel que isobornyle, cyclohexyle,
      - un groupe aryle en C₃ à C₂₀ tel que phényle,
      - un groupe aralkyle en C₄ à C₃₀ (groupe alkyle en C₁ à C₈) tel que 2-phényléthyle ; benzyle,
      - un groupe hétérocyclique de 4 à 12 chaînons contenant un ou plusieurs hétéroatomes choisis parmi O, N, et S, le cycle étant aromatique ou non,
      - un groupe hétérocyclylalkyle (alkyle de 1 à 4 C), tel que furfurylméthyle ou tétrahydrofurfurylméthyle,
lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si (R₄R₅) où R₄ et R₅ identiques ou différents représentent un groupe alkyle en C₁ à C₆, ou un groupe phényle.

Des exemples de monomères vinyliques sont le vinylcyclohexane, et le styrène.

Des exemples d'esters de vinyle sont : l'acétate de vinyle le propionate de vinyle, le butyrate de vinyle, l'éthylhexanoate de vinyle , le néononanoate de vinyle ; et le néododécanoate de vinyle.

Parmi les éthers de vinyle on trouve par exemple le vinyl méthyl ether, le vinyl éthyl éther, et le vinyl isobutyl éther ;
- Les monomères (méth)acryliques ou (méth)acrylamides ou vinyliques à groupe fluoré ou perfluoré, tels que le méthacrylate d'éthyl perfluorooctyle ;
- Les monomères (méth)acryliques ou vinyliques siliconés, tels que le méthacryloxypropyltris(triméthylsiloxy)silane, l'acryloxypropylpolydiméthylsiloxane, ou les (méth)acrylamides siliconés.

Les monomères, préférés, pour la séquence B, sont ceux dont la température de transition vitreuse Tg de l'homopolymère correspondant se situe dans la plage de -55°C à +55°C, tels que : méthacrylate d'hydroxyéthyle (55°C), le méthacrylate d'isobutyle (53°C), l'acrylate de n-hexyle (Tg=45°C), l'acrylate de t-butyle (Tg=50°C) , l'acétate de vinyle (Tg=23°C) , le méthacrylate de butyle (Tg=20°C), l'acrylate de cyclohexyle (19°C), l'acrylate d'hydroxyéthyle (15°C),l'acrylate de méthyle (Tg=10°C),le méthacrylate d'éthoxyéthyle (Tg=0°C), le méthacrylate de n-hexyle (-5°C), ), le butyrate de vinyle (-5°C), l'acrylate d'éthyle (Tg=-24°C), l'acrylate d'isobutyle (-24°C), le vinylméthyl ether (-34°C), l'acrylate de méthoxyéthyle (-33°C), l'acrylate de n-hexyle (-45°C), l'acrylate de n-butyle (Tg=-54°C),l'acrylate d'éthylhexyle (Tg=-50°C), le méthacrylate de POE (n=8 à 10) (Tg=-55°C) .

Parmi les autres monomères préférés dont la Tg varie en dehors de la plage -55 à 55°C, on peut citer par exemple, les monomères suivants : acrylate de t-butylcyclohexyle, acrylate de t-butylbenzyle, acrylate de furfuryle et acrylate d'isobornyle ; méthacrylate de méthyle, méthacrylate d'éthyle, méthacrylate de cyclohexyle, méthacrylate de t-butylcyclohexyle, méthacrylate de t-butylbenzyle, et méthacrylate d'isobornyle ;
N-butylacrylamide, N-t-butylacrylamide, N-isopropylacrylamide, N,N-diméthylacrylamide et N,N-dibutylacrylamide ;
vinylcyclohexane, styrène ;
acrylate d'hexyle, acrylate d'octyle, acrylate de lauryle, acrylate d'isooctyle, acrylate d'isodécyle ;
méthacrylate d'éthylhéxyle, méthacrylate d'octyle, méthacrylate de lauryle, méthacrylate d'isooctyle, méthacrylate d'isodécyle ;
néononanoate de vinyle, néododécanoate de vinyle ;
N-octylacrylamide.

La séquence B de Tg globale allant de -55°C à +55°C peut donc, de préférence, être constituée en totalité ou en partie par un ou des monomères, cités ci-dessus, dont la température de transition vitreuse de l'homopolymère correspondant se situe dans la plage de -55°C à +55°C. Si la séquence B est constituée en totalité par de tels monomères, il peut s'agir d'un homopolymère ou bien d'un copolymère qui est alors constitué d'au moins deux monomères, de façon à ce que la séquence B présente globalement une seule Tg et non, par exemple, deux Tg différentes, séparées : Tg₁ et Tg₂ qui correspondraient chacune à la température de transition vitreuse des homopolymères correspondant à chacun des deux monomères formant la séquence B.

La séquence B peut n'être constituée qu'en partie par un ou des monomères dont la température de transition vitreuse de l'homopolymère correspondant se situe dans la plage de -55°C à +55°C.

Dans ce cas, la séquence de température de transition vitreuse comprise entre -55°C et +55°C peut, en plus des monomères indiqués ci-dessus, et dont la température de transition vitreuse Tg de l'homopolymère correspondant est dans la plage de -55°C à +55°C, par exemple inférieure ou égale à 20°C, comprendre un ou plusieurs autres monomères. Ces monomères sont des monomères dont la température de transition vitreuse Tg de l'homopolymère correspondant se situe en dehors de la plage -55°C, +55°C.

Ce ou ces monomères sont, bien sûr, choisis de façon à ce que la Tg globale de la séquence se trouve dans la plage indiquée plus haut.

La séquence B peut aussi être un copolymère, constitué en totalité par des monomères, dont la température de transition vitreuse Tg de l'homopolymère correspondant se situe en dehors de la plage allant de -55°C à +55°C.

Ces monomères sont bien sûr choisis de façon à ce que la séquence B ait globalement une température de transition vitreuse se situant dans la plage de -55°C à +55°C.

Les monomères particulièrement préférés dont la Tg varie dans la plage -55 à 55°C parmi tous ceux cités-ci-dessus sont choisis parmi l'acrylate de n-hexyle (Tg=45°C), l'acrylate de t-butyle (Tg=50°C) l'acétate de vinyle (Tg=23°C) ,le méthacrylate de butyle (Tg=20°C), l'acrylate d'hydroxyéthyle (15°C), l'acrylate de cyclohexyle (19°C) , l'acrylate de méthyle (Tg=10°C), le butyrate de vinyle (-5°C), le vinylméthyl ether (-34°C), l'acrylate de n-butyle (Tg=-54°C), ,l'acrylate d'éthylhexyle (Tg=-50°C), l'acrylate d'isobutyle (-24°C), l'acrylate de méthoxyéthyle (-33°C), le méthacrylate de n-hexyle (-5°C), le méthacrylate de POE (n=8 à 10) (Tg=-55°C), l' acrylate hydroxypropyle.

De préférence, un ou plusieurs monomères parmi les monomères qui constituent la ou les séquences B sont choisis parmi les monomères hydrophiles. De préférence, un ou plusieurs parmi les séquences B autres que la séquence A sont hydrophiles et contiennent des monomères hydrophiles.

Avantageusement, le copolymère, selon l'invention, comprend au moins une séquence hydrophile qui comprend des monomères hydrophiles. Cette ou ces séquence(s) peut(vent) être la séquence qui comprend une proportion de 52 à 100% en poids de monomère éthylénique à cycle lactame, tel qu'un vinyllactame, et/ou une ou plusieurs des autres séquences qui constituent le polymère, par exemple une ou plusieurs des séquences B.

La séquence hydrophile peut être définie comme étant une séquence hydrosoluble ou hydrodispersible.

Le polymère formant la séquence est hydrosoluble s'il est soluble dans l'eau, à raison d'au moins 5% en poids, à 25°C.

Le polymère formant la séquence est hydrodispersible, s'il forme à une concentration de 5 %, à 25 °C, une suspension stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.

La séquence hydrophile est, de préférence, une séquence dont la température de transition vitreuse est élevée, par exemple de 0 à 250°C, de préférence de 50 à 200°C.
- Des exemples de monomères hydrophiles incluent les monomères cationiques, les monomères anioniques et les monomères non ioniques :
- Des exemples de monomères cationiques sont :
   - la 2-vinylpyridine (Tg : 104°C) ;
   - la 4-vinylpyridine (Tg : 142°C) ;
   - le (méth)acrylate de diméthylaminoéthyle (Tg : 19°C);
   - le (méth)acrylate de diéthylaminoéthyle
   - le (méth)acrylamide de diméthylaminopropyle ;
et les formes salifiées ou quaternisées de ceux-ci, qu'il s'agisse de sels d'acides minéraux, tels que l'acide sulfurique ou l'acide chlorhydrique, ou de sels d'acides organiques.

Ces acides organiques peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle.

Un exemple d'acide à groupe alkyle est l'acide acétique CH₃COOH.

Un exemple de polyacide est l'acide téréphtalique.

Des exemples d'hydroxyacides sont l'acide citrique et l'acide tartrique.
- Des exemples de monomères anioniques sont :
   - l'acide acrylique, l'acide méthacrylique, l'acide crotonique, l'anhydride maléique, l'acide itaconique, l'acide fumarique, l'acide maléique ;
   - l'acide styrènesulfonique, l'acide 2-acrylamido 2-methylpropanesulfonique, l'acide vinylbenzoïque, l'acide vinylphosphorique, le (méth)acrylate de sulfopropyle, et les sels de ceux-ci.
   Le neutralisant peut être une base minérale, telle que LiOH, NaOH, KOH, Ca(OH)₂, NH₄OH ; une base organique. Il peut s'agir d'une alkylamine primaire , secondaire ou tertiaire, telle que la triéthylamine, ou la butylamine. Cette alkylamine primaire , secondaire ou tertiaire, peut en outre comporter des azotes ou oxygènes et donc comporter par exemple une fontion alcool par exemple l'amino-2-méthyl-2-propanol, la triéthanolamine.
- Des exemples de monomères non-ioniques sont :
   - les carboxybétaïnes ou sulfobétaïnes éthyléniques obtenues, par exemple par quaternisation de monomères à insaturation éthylénique comportant une fonction amine par des sels d'acide carboxylique à halogène mobile, par exemple, le chloroacétate de sodium, ou par des sulfones cycliques, par exemple la propanesulfone ;
   - les (méth) acrylates ou (métha) crylamides d'hydroxyalkyle dont le groupe alkyle a de 2 à 4 atomes de C, en particulier le (méth)acrylate d'hydroxyéthyle ou d'hydroxypropyle ;
   - les (méth)acrylates ou (métha)crylamides d' alcoxy (C₁₋₄) alkyle (C₁₋₄) tel que méthoxyéthyle, éthoxyéthyle et méthoxypropyle,
   - les (méth)acrylates ou (métha)crylamides à groupe - (OC₂H₄)ₘ-OR"', avec m = 5 à 150 et, R''' = H ou alkyle de C₁ à C₄, par exemple -POE- méthoxy ;-POE-OH ;
   - les vinyllactames,
   - et les (méth)acrylates de polysaccharide comme l'acrylate de saccharose.
Les vinyllactames ne peuvent, toutefois, constituer qu'une proportion minoritaire de la séquence B.

Il est à noter que même si le copolymère comprend une séquence hydrophile, le copolymère global n'est pas forcément hydrophile.

Les copolymères éthyléniques, séquences, linéaires selon l'invention sont choisis parmi :
- les copolymères biséquencés (AB),
- les copolymères triséquencés (ABA, BAB, ABC, ACB), avec C différent de A ou B,
- les copolymères multiséquencés ayant plus de trois séquences : (AB) ₙ, (ABA) ₙ, (BAB) ₙ, (ABC) ₙ, avec C différents de A ou B, ou les copolymères multiséquencés ayant plus de trois séquences différentes, de type ABCD.

Les copolymères selon l'invention peuvent être préparés par polymérisation par voie anionique.

De préférence, toutefois, les copolymères, selon l'invention, sont dans un premier mode obtenus par polymérisation radicalaire contrôlée et, de manière particulièrement préférée, les copolymères selon l'invention peuvent être obtenus par une polymérisation par « ATRP » particulière qui est la technique dite « Reverse ATRP », ou par la technique « RAFT », mais les polymères selon l'invention peuvent aussi, selon un second mode, être obtenus par polymérisation radicalaire classique.

### Premier mode

Les copolymères blocs ou séquences selon l'invention sont de préférence obtenus par polymérisation radicalaire contrôlée, décrite notamment dans "New Method of Polymer Synthesis", Blackie Académie & Professional, Londres, 1995, volume 2, page 1.

La polymérisation radicalaire contrôlée permet de réduire les réactions de désactivation de l'espèce radicalaire en croissance, en particulier l'étape de terminaison, réactions qui, dans la polymérisation radicalaire classique, interrompent la croissance de la chaîne polymérique de façon irréversible et sans contrôle.

Afin de diminuer la probabilité des réactions de terminaison, il a été proposé de bloquer de façon transitoire et réversible, l'espèce radicalaire en croissance, en formant des espèces actives dites "dormantes" sous forme de liaison de faible énergie de dissociation.

Ainsi, la polymérisation peut être effectuée selon la technique de transfert d'atome (Atom Transfer Radical Polymerization ou « ATRP », en anglais), ou par réaction avec un nitroxyde, ou bien encore selon la technique de "*reversible addition-fragmentation chain transfer*" (« RAFT ») ou enfin par la technique de « ATRP » inverse, dite « reverse ATRP ».

La technique de polymérisation radicalaire par transfert d'atomes, aussi connue sous l'abréviation ATRP, consiste à bloquer l'espèce radicalaire en croissance sous forme de liaison de type C-halogénure (en présence de complexe métal/ligand). Ce type de polymérisation se traduit par un contrôle de la masse des polymères formés et par un faible indice de dispersité des masses.

D'une manière générale, la polymérisation radicalaire par transfert d'atomes s'effectue par polymérisation d'un ou de plusieurs monomères polymérisables par voie radicalaire, en présence :
- d'un amorceur ayant au moins un atome d'halogène transférable, ;
- d'un composé halogéné comprenant un métal de transition susceptible de participer à une étape de réduction avec l'amorceur et une chaîne polymérique "dormante", celui ci sera dénommé « agent de transfert de chaîne » ; et
- d'un ligand pouvant être choisi parmi les composés comprenant un atome d'azote (N), d'oxygène (O), de phosphore (P) ou de soufre (S), susceptibles de se coordonner par une liaison σ audit composé comprenant un métal de transition ; la formation de liaisons directes entre ledit composé comprenant un métal de transition et le polymère en formation étant évitées.

L'atome d'halogène est de préférence un atome de chlore ou de brome.

Ce procédé est en particulier décrit dans la demande WO 97/18247 et dans l'article de Matyjasezwski *et al.* publié dans *JACS,* 117, page 5614 (1995).

La technique de polymérisation radicalaire par réaction avec un nitroxyde consiste à bloquer l'espèce radicalaire en croissance sous forme de liaison de type C-O-NRₐR_{b} où Rₐ et R_{b} peuvent être, indépendamment l'un de l'autre, un radical alkyle ayant de 2 à 30 atomes de carbone ou formant l'un et l'autre, avec l'atome d'azote, un cycle ayant de 4 à 20 atomes de carbone, comme par exemple un cycle 2,2,6,6-tétraméthylpipéridinyle. Cette technique de polymérisation est notamment décrite dans les articles « Living free radical polymerization : a unique technique for préparation of controlled macromolecular architectures » CJ Hawker; Chem. Res. , 1997,30,373-82 et "Macromolecular engineering via living free radical polymerizations" publié dans *Macromol. Chem. Phys.* 1998, vol. 199, pages 923 - 935, ou bien encore dans la demande WO-A-99/03894.

La technique de polymérisation RAFT (*reversible addition-fragmentation chain transfer*) consiste à bloquer l'espèce radicalaire en croissance sous forme de liaison de type C-S. On utilise pour cela des composés dithio comme les dithioesters (-C(S)S-), tels que les dithiobenzoates, les dithiocarbamates (-NC(S)S-) ou les dithiocarbonates (-OC(S)S-) (xanthates). Ces composés permettent de contrôler la croissance de la chaîne d'une large gamme de monomères. Toutefois, les dithioesters inhibent la polymérisation des esters vinyliques, tandis que les dithiocarbamates sont très faiblement actifs vis-à-vis des méthacrylates, ce qui limite dans une certaine mesure l'application de ces composés. Cette technique est notamment décrite dans la demande WO-A-98/58974 de RHODIA et dans l'article "A more versatile route to block copolymers and other polymers of complexe architecture by living radical polymerization : the RAFT process", publié dans *Macromolecules,* 1999, volume 32, pages 2 071-2 074. La demande WO-A-98/58974 déjà citée et la demande WO-A-99/31144 de CSIRO ont trait à l'utilisation de dithiocarbamates en tant que réactifs « RAFT ». En utilisant ces dithiocarbamates divers monomères sont polymérisés dont l'acétate de vinyle.

Les avantages principaux de la technique « RAFT » sur la technique « ATRP » sont qu'elle ne nécessite pas de catalyseur métallique et également que des amorceurs de radicaux classiques peuvent être utilisés pour amorcer la réaction.

Dans le cas de la polymérisation radicalaire contrôlée par exemple de la N-vinylpyrrolidone (NVP), les agents de transfert de chaîne préférés utilisés dans le cas de la polymérisation « RAFT » sont les dérivés de diphényldithiocarbamate.

En faisant varier le rapport de la concentration en monomère sur la concentration en agent de transfert de chaîne, la masse moléculaire du polymère peut être modifiée.

La polymérisation se déroule en plusieurs étapes selon le schéma général :
a- Dans une première étape, on effectue la polymérisation du premier monomère ou mélange de monomères pour former un macroamorceur,
b- Les polymères purifiés par précipitation sont séchés sous vide,
c- Ensuite, dans la deuxième étape, on réalise la polymérisation de la seconde séquence constituée par un monomère ou un mélange de monomères, à l'extrémité du macroamorceur (formé à l'étape a).

Les étapes b et c sont répétées autant de fois que nécessaire suivant le nombre de séquences.

Un procédé particulièrement privilégié est le procédé de polymérisation dit « ATRP » inverse (« Reverse ATRP » en anglais) à ne pas confondre avec le procédé de ATRP classique décrit plus haut.

Dans ce procédé de « Reverse ATRP », l'amorçage est réalisé de façon classique par un amorceur capable de donner des radicaux, tels que par exemple l'azobisisobutyronitrile (AIBN) ou un peroxyde.(et non plus à l'aide d'un amorceur spécifique).

La présence d'halogénures de métal, tel que CuBr₂ et d'un ligand permet le contrôle de la polymérisation par « capture » réversible des radicaux formés.

Les ligands préférés sont des molécules à base d'amine en particulier la tris (diméthylaminoéthyl) amine (Me₆TREN). Un bon contrôle de la polymérisation est observé en particulier avec le système AIBN/CuBr₂/Me₆TREN.

L'autre procédé privilégié est le procédé de polymérisation dit « RAFT »

### Deuxième mode

Les polymères blocs ou séquencés selon l'invention peuvent également être obtenus en utilisant la technique de polymérisation radicalaire classique en effectuant la coulée des monomères de façon séquencée. Dans ce cas, seul le contrôle de la nature des séquences est possible (pas de contrôle des masses) .

Il s'agit de polymériser dans un premier temps un monomère M1 dans un réacteur de polymérisation; de suivre, par cinétique, sa consommation dans le temps puis quand M1 est consommé à environ 95% alors d'introduire un nouveau monomère M2 dans le réacteur de polymérisation.

On obtient ainsi un polymère de structure bloc de type M1-M2.

De manière plus précise, on décrit un premier procédé de préparation d'un copolymère tel que décrit ci-dessus comprenant au moins une séquence A et au moins une séquence B et éventuellement une ou plusieurs autres séquences différentes des séquences A et B, dans lequel la polymérisation est réalisée par la technique dite de polymérisation radicalaire à transfert d'atomes inverse (« Reverse ATRP ») comprenant les étapes successives suivantes :
a) on polymérise le monomère ou les monomères à partir desquels est préparée la séquence A en présence d'un agent de transfert de chaîne tel qu'un halogénure de métal de transition, d'un amorceur de radicaux, et d'un ligand, et en présence ou non d'un solvant, moyennant quoi, on obtient un macroamorceur ou précurseur fonctionnel capable d'amorcer une polymérisation car comportant à ses extrémités la fonction agent de transfert de chaîne ;
b) on polymérise le monomère ou les monomères à partir desquels est préparée la séquence B à l'extrémité dudit macroamorceur ou précurseur en présence d'un agent de transfert de chaîne tel qu'un halogénure de métal de transition, et d'un ligand, et en présence ou non d'un solvant, moyennant quoi on obtient un copolymère biséquencé de structure A-b-B ;
c) on répète, éventuellement, l'étape b) avec le ou les monomères à partir desquels sont préparées la ou les autres séquences différentes des séquences A et B, moyennant quoi on obtient un copolymère triséquencé ou multiséquencé.

De façon à obtenir des copolymères triséquencés symétriques, il est possible d'utiliser des amorceurs difonctionnels.

Avantageusement, les « agents de transfert de chaîne » sont choisis parmi les halogénures de métaux à l'état d'oxydation le plus élevé, de préférence parmi CuBr₂, CuCl₂, FeCl₂P (phényl) ₃ ; FeCl₃, et RuCl₂P (phényl)₃.

Avantageusement, les amorceurs de polymérisation radicalaire sont choisis parmi :
- les composés azoïques, tels que le 2,2'-azobisisobutyronitrile (AIBN), le 2,2'-azobis(2-butanenitrile), le 4,4'-azobis(4-acide pentanoïque), le 1,1'-azobis(cyclohexanecarbonitrile), le 2-(t-butylazo)-2-cyanopropane, le 2,2'-azobis[2-méthyl-N-(1,1)-bis(hydroxyméthyl)-2-hydro xyéthyl]propionamide, le 2,2'-azobis(2-méthyl-N-hydroxyéthyl)propionamide, le dichlorure de 2,2'-azobis(N,N'-diméthylèneisobutyramidine), le dichlorure de 2,2'-azobis(2-amidinopropane), le 2,2'-azobis(N,N'-diméthylèneisobutyramide), le 2,2'-azobis(2-méthyl-N-[1,1-bis(hydroxyméthyl)-2-hydrox yéthyl]propionamide), le 2,2'-azobis(2-méthyl-N-[1,1-bis(hydroxyméthyl)éthyl] propionamide), le 2,2'-azobis(2-méthyl-N-(2-hydroxyéthyl)propionamide), le 2,2'-azobis(isobutyramide)dihydrate ;
- les péroxydes d'hydrogène tels que l'hydropéroxyde de butyle tertiaire, l'hydropéroxyde de cumène, le t-butyl-peroxyacétate, le t-butylperoxybenzoate, le t-butylperoxyoctoate, le t-butylperoxynéodécanoate, le t-butylpéroxyisobutarate, le peroxyde de lauroyle, le t-amylperoxypivalate,le t-butylperoxypivalate, le peroxyde de dicumyle, le peroxyde de benzoyle,
- les persulfates alcalins tels que le persulfate de potassium, le persulfate d'ammonium ;
- les systèmes redox comportant des combinaisons, telles que :
   - les mélanges de peroxyde d'hydrogène, d'alkyle, peresters, percarbonates et similaires et de n'importe lequel des sels de fer, de sels titaneux, formaldéhyde sulfoxylate de zinc ou formaldéhyde sulfoxylate de sodium, et des sucres réducteurs,
   - les persulfates, perborate ou perchlorate de métaux alcalins ou d'ammonium en association avec un bisulfite de métal alcalin, tel que le métabisulfite de sodium, et des sucres réducteurs,
   - les persulfates de métal alcalin en association avec un acide arylphosphinique, tel que l'acide benzène phosphonique et autres similaires, et des sucres réducteurs.

Avantageusement, les ligands sont choisis parmi les ligands tétradentate, tels que le 1,1,4,7,10,10 hémaméthyltriéthylènetétramine (HMTETA), le 1,4,8,11 tétraazacyclotétradécane (cyclame), le 1,4,8,11 tétraméthyl-1,4,8,11-tétraazacyclotétradécane (Me₄ cyclame), et la tris(diméthylaminoéthyl)amine (Me₆TREN) ; les ligands hexadentate, tels que la tétra 2-pyridyl pyrazine (TPPY), la N,N,N',N' tétrabis(2-pyridyl méthyl)éthylène diamine (TPMEDA) ; et la triphénylphosphine (TPP).

Avantageusement, les solvants sont choisis parmi le dioxane, le tétrahydrofuranne (THF), la N-méthylpyrrolidone, l'eau, et leurs mélanges.

Les « agent de transfert de chaîne » amorceurs, ligands, solvants peuvent être identiques ou différents dans l'étape a) et l'étape b), mais de préférence, ils sont identiques. De préférence encore dans la première et la deuxième étapes a) et b), l'« agent de transfert de chaîne » est CuBr₂, l'amorceur est AIBN, le ligand est Me₆TREN, et le solvant est le dioxane.

Ce premier procédé peut permettre de préparer l'un quelconque parmi les copolymères de l'invention, mais il s'applique particulièrement à la préparation d'un copolymère dans lequel la séquence A est un homopolymère issu d'un monomère choisi parmi la N-vinylpyrrolidone et la N-vinylcaprolactame et la séquence B est un homopolymère issu d'un monomère choisi parmi l'acrylate de méthyle, l'acrylate de t-butyle et le méthacrylate de méthyle.

On décrit en outre un second procédé de préparation d'un copolymère tel que décrit ci-dessus comprenant au moins une séquence A et au moins une séquence B et éventuellement une ou plusieurs autres séquences différentes des séquences A et B, dans lequel la polymérisation est réalisée par la technique dite de polymérisation RAFT « Réversible Addition-Fragmentation Chain Transfer » comprenant les deux étapes successives suivantes :
a) on polymérise le ou les monomères à partir desquels est préparée la séquence A en présence d'un agent de transfert de chaîne, et d'un amorceur, dans un solvant ou non, moyennant quoi on obtient un macroamorceur ou précurseur comportant à ses extrémités la fonction agent de transfert de chaîne ;
b) on polymérise le ou les monomères à partir desquels est préparée la séquence B à l'extrémité dudit macroamorceur ou précurseur, en présence d'un amorceur dans un solvant ou non, moyennant quoi on obtient un copolymère A-b-B ;
c) on répète, éventuellement, l'étape b) avec le ou les monomères à partir desquels sont préparées la ou les autres séquences différentes des séquences A et B, moyennant quoi on obtient un copolymère triséquencé ou multiséquencé.

Avantageusement, les agents de transfert de chaîne sont choisis parmi les dithioesters (-C(S)S-), tels que les dithiobenzoates, les dithiocarbamates (-NC(S)S-) et les dithicarbonates (-OC(S)S-) (xanthates).

De préférence, les agents de transfert de chaîne sont choisis parmi le diphényl dithiocarbamate de malonate de diéthyle (DPCM), le diphényldithiocarbamate d'acétate de fluoroéthyle (DPFEM) et le xanthate de formule C₂H₅OC (S) SCH (CH₃) COOCH₃.

Avantageusement, les amorceurs sont choisis parmi les composés déjà cités ci-dessus pour le premier procédé.

Avantageusement, les solvants sont choisis parmi le dioxane, le tétrahydrofuranne, la N Methylpyrrolidone, l'eau ou mélange.

Les amorceurs, agents de transfert de chaîne et solvants peuvent être identiques ou différents dans l'étape a) et l'étape b). De préférence, ils sont identiques.

De préférence encore dans la première et la deuxième étapes a) et b), l'amorceur est AIBN, l'agent de transfert de chaîne est le diphényl dithiocarbamate de malonate de diéthyle (DPCM), le diphényldithiocarbamate d'acétate de fluoroéthyle (DPFEM) ou le xanthate de formule C₂H₅OC (S) SCH (CH₃) COOCH₃ suivant la nature des monomères à polymériser, et le solvant est le dioxane.

Ce second procédé peut permettre de préparer l'un quelconque parmi les copolymères de l'invention, mais il s'applique particulièrement à la préparation d'un copolymère dans lequel la séquence A est un homopolymère issu d'un monomère choisi parmi la N-vinylpyrrolidone et la N-vinylcaprolactame et la séquence B est un homopolymère issu d'un monomère choisi parmi l'acrylate de méthyle, l'acrylate de t-butyle et le méthacrylate de méthyle.

L'invention concerne également les compositions cosmétiques ou pharmaceutiques comprenant le copolymère de structure spécifique, tel qu'il a été décrit ci-dessus.

Généralement, ces compositions contiennent de 0,1 à 60 % en poids, de préférence de 0,5 à 50 % en poids, et de préférence encore de 1 à 40 % en poids du copolymère selon l'invention.

Ces compositions cosmétiques, selon l'invention, comprennent, outre lesdits polymères, un milieu physiologiquement acceptable, c'est-à-dire un milieu compatible avec les matières kératiniques, comme la peau, les cheveux, les cils, les sourcils et les ongles.

De manière générale, il faut considérer que l'ensemble de la composition est physiologiquement acceptable.

Ledit milieu, physiologiquement acceptable, comprend généralement un solvant approprié, physiologiquement acceptable, dans lequel le copolymère, selon l'invention, se trouve sous forme dissoute ou dispersée.

La composition peut ainsi comprendre, en tant que solvant formant une phase hydrophile, de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols. La phase hydrophile peut, en outre, contenir des éthers en C₂. L'eau ou le mélange d'eau et de solvants organiques hydrophiles peut être présent dans la composition selon l'invention en une teneur allant de 0 % à 90 % (notamment 0,1 % à 90 %) en poids, par rapport au poids total de la composition, et de préférence de 0 % à 60 % en poids (notamment 0,1 % à 60 % en poids).

La composition peut également comprendre une phase grasse, notamment constituée de corps gras liquides à température ambiante (25°C en général) et/ou de corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.

Comme corps gras liquides à température ambiante, appelés souvent huiles, utilisables dans l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent être présentes en une teneur allant de 0,01 à 90 %, et mieux de 0,1 à 85 % en poids, par rapport au poids total de la composition.

La composition selon l'invention peut également comprendre un ou plusieurs solvants organiques, cosmétiquement acceptables (tolérance, toxicologie et toucher acceptables). Ces solvants peuvent être présents généralement en une teneur allant de 0 à 90 %, de préférence de 0,1 à 90 %, de préférence encore de 10 à 90% en poids, par rapport au poids total de la composition, et mieux de 30 à 90 %.

Comme solvants utilisables dans la composition de l'invention, on peut citer les esters de l'acide acétique comme l'acétate de méthyle, d'éthyle, de butyle, d'amyle, de méthoxy-2-éthyle, l'acétate d'isopropyle ; les cétones comme la méthyléthylcétone, la méthylisobutylcétone ; les hydrocarbures comme le toluène, le xylène, l'hexane, l'heptane ; les aldéhydes ayant de 5 à 10 atomes de carbone ; les éthers ayant au moins 3 atomes de carbones ; et leurs mélanges.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0 à 50 % en poids de cires, par rapport au poids total de la composition et mieux de 1 à 30 % en poids.

Le polymère peut être associé à un ou des agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Les colorants hydrosolubles sont par exemple le jus de betterave, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut également contenir des ingrédients couramment utilisés en cosmétique, tels que les vitamines, les épaississants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents antipelliculaires, les agents propulseurs, les polymères hydrosolubles, liposolubles ou en dispersion dans l'eau ou dans une phase grasse, filmogènes ou non, ou leurs mélanges.

Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 s⁻¹, après 10 minutes de mesure en géométrie cône/plan). La composition peut être anhydre, par exemple il peut s'agir d'une pâte anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage comme les produits pour le teint (fonds de teint), les fards à joues ou à paupières, les produits pour les lèvres, les produits anti-cernes, les blush, les mascaras, les eye-liners, les produits de maquillage des sourcils, les crayons à lèvres ou à yeux, les produits pour les ongles, tels que les vernis à ongles, les produits de maquillage du corps, les produits de maquillage des cheveux (mascara ou laque pour cheveux).

La composition selon l'invention peut être également un produit capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray.

Le polymère selon l'invention lorsqu'il est utilisé dans les produits capillaires tels que les produits pour le maintien de la coiffure ou la mise en forme des cheveux permet d'éviter le poudrage " flaking ".

Les solutions peuvent être conditionnées sous diverses formes : gels, lotions, par exemple, et notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsque l'on souhaite obtenir un spray, une mousse pour la fixation ou le traitement des cheveux.

On a vu plus haut que le polymère selon l'invention était particulièrement adapté à une mise en oeuvre dans de telles conditions car il présentait une grande compatibilité avec les agents propulseurs utilisés notamment dans les récipients aérosols. Le ou les agents propulseurs peuvent être choisis parmi le diméthyléther, les alcanes en C₃₋₅, tels que le propane, le n-butane et l'isobutane ; le 1,1-difluoroéthane, les mélanges de diméthyléther et d'alcanes en C₃₋₅, et les mélanges de 1,1-difluoroéthane et de diméthyléther et/ou d'alcanes en C₃₋₅.

L'invention va maintenant être décrite, en référence aux exemples suivants, donnés à titre illustratif et non limitatif.

### Exemples

Dans les exemples suivants, on prépare des polymères selon l'invention, par la technique de polymérisation radicalaire par transfert d'atomes inverse, aussi connue sous l'abréviation « Reverse ATRP », ou par la technique de polymérisation « RAFT » (Reverse Addition - Fragmentation Chain Transfer).

De manière générale, la formation de blocs dans le cas du procédé « ATRP » inverse et du procédé « RAFT » se déroule en plusieurs étapes.

Ainsi dans le cas d'un copolymère dibloc, on procédera par exemple de la manière suivante :
- a/ La première étape consiste en une polymérisation du premier monomère pour former le macroamorceur ou précurseur.
- b/ La seconde étape consiste en la polymérisation du second monomère à l'extrémité du macroamorceur pour former le dibloc.

Entre les deux étapes, une étape de purification peut être nécessaire, en particulier dans le cas de la polymérisation dite « Reverse ATRP ».

On décrira tout d'abord le mode opératoire général utilisé pour la réalisation des exemples, pour chacun des procédés : procédé 1 (polymérisation « Reverse ATRP ») et procédé 2 (polymérisation « RAFT ») .

### 1. Procédé 1 : « ATRP » inverse

On réalise le procédé de « Reverse ATRP », de préférence avec la tris(diméthylaminoéthyl)amine (TREN Me₆) en tant que ligand, en utilisant CuBr₂ et en mettant en oeuvre l'AIBN en tant qu'amorceur.

### 1. 1. Matières premières

- Le monomère éthylénique à cycle lactame, tel que la N-vinylpyrrolidone (VP), acquis auprès de la Société ALDRICH® , est distillé sous vide et stocké sous azote à 0°C avant utilisation.
- Les autres monomères, tels que acrylate de tertiobutyle, méthacrylate de méthyle, acrylate de méthyle, acquis auprès de la Société ALDRICH® sont séchés sur hydrure de calcium et distillés sous vide.
- L'amorceur azobisisobutyronitrile (AIBN) est recristallisé dans le méthanol.
- Le CuBr₂ (99,9 %), le CuBr, la poudre de cuivre (99 %) sont acquis auprès de la Société ALDRICH® et utilisés tels qu'ils sont fournis.
- La tris (diméthylaminoéthyl) amine (TREN Me₆) est synthétisée selon les procédures décrites dans la littérature, par exemple dans le document de Matyjaszewski ACS Symp Ser 2000 ; 760 ; 207.
- Tous les solvants : THF, dioxane, et DMF sont séchés par distillation sur CaH₂ avant leur utilisation.

### 1. 2. Polymérisation

La procédure générale est la suivante :

### 1. 2. 1. Première étape

### Formation du macroamorceur ou précurseur

CuBr₂ et Me₆ TREN sont mis dans un ballon et on leur additionne du dioxane.

La solution est agitée durant 30 min. à 25°C.

Le monomère est additionné puis l'amorceur AIBN.

Le système est soumis à trois cycles de séchage par vide/argon. La solution est ensuite chauffée dans un bain thermostaté.

La viscosité augmente. Après réaction pendant la durée voulue, le système est refroidi à température ambiante.

Le polymère macroamorceur est séparé par précipitation : il est dilué dans le chloroforme puis précipité dans le diéthyléther, cette opération est répétée deux fois. Le complexe de cuivre est séparé du polymère dissous dans le chloroforme par passage sur colonne neutre. La solution de polymère est alors évaporée.

### 1. 2. 2. Deuxième étape

### Polymérisation de la seconde séquence à l'extrémité du macroamorceur

Le macroamorceur obtenu dans la première étape est additionné à une solution de CuBr et de Me₆TREN dans le dioxane sous atmosphère inerte d'azote.

La quantité requise de manomère(s) constituant la seconde séquence est additionnée. La solution est chauffée. Après le temps désiré, le système est refroidi à température ambiante.

Le polymère est séparé par précipitation : il est dilué dans le chloroforme puis précipité dans le diéthyl éther, cette opération est répétée deux fois. Le complexe de cuivre est séparé du polymère dissous dans le chloroforme par passage sur colonne neutre. La solution de polymère est évaporée.

Le polymère est séché.

Le polymère isolé est généralement une poudre blanche.

### 2. Procédé 2 : « RAFT »

On réalise la polymérisation de monomères éthyléniques à cycle lactame, par exemple de vinylactames avec les composés suivants en tant qu'agents de transfert de chaîne :
- diphényldithiocarbamate de diéthylmalonate (DPCM) : 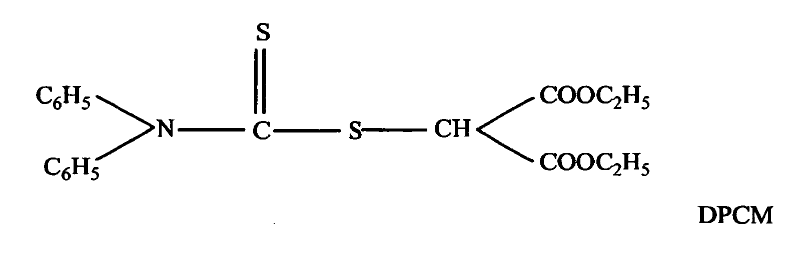
- diphényl dithiocarbamate de fluoroacétate d'éthyle (DPFEM) 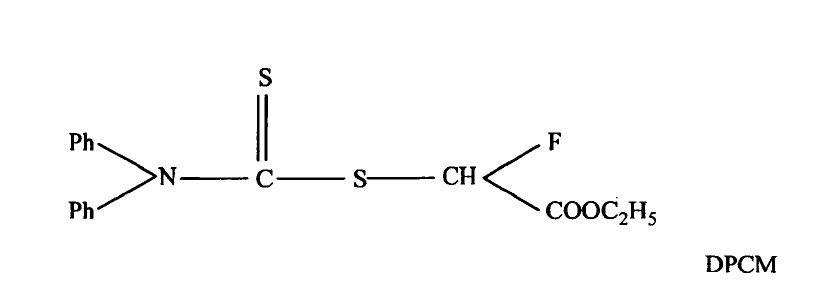
- xanthate: C₂H₅ O C(S)S C H (CH₃) COOCH₃

### Matières premières

- Le monomère éthylénique à cycle lactame tel que la N-vinylpyrrolidone (VP) ou la N-vinylcaprolactame (VCap) est acquis auprès de la Société ALDRICH® et est distillé sous vide avant utilisation.
- Les autres monomères, tel que acrylate de teriobutyle, acrylate de méthyle, méthacrylate de méthyle sont acquis auprès de la Société ALDRICH® et sont séchés sur hydrure de calcium et distillés sous vide.
- L'AIBN est acquis auprès de la Société ALDRICH® et est recristallisé dans le méthanol.
- La diphénylamine, le CS₂, et le bromodiéthylmalonate sont acquis auprès de la Société ALDRICH® et sont utilisés tels qu'ils sont reçus.
- Les solvants tels que le dioxane sont séchés par distillation sur CaH₂ avant utilisation.

### 2. 1. Synthèse de l'agent de transfert de chaîne.

### 2.1.1. : Synthèse du diphényldithiocarbamate de diéthyl malonate (DPCM)

Le diphényldithiocarbamate diéthylmalonate (DPCM) est synthétisé à partir de la diphénylamine et du malonate de bromodiéthyle de la manière suivante :

A une solution de 0,625 g de NaH (purifié par lavage avec de l'hexane sec) dans 5 ml de THF (sec), on ajoute à 0°C, 1,69 g de diphénylamine (10 mmol) dans 10 ml de DMSO et 5 ml de THF. Le mélange réactionnel est agité pendant 1,5 heures pour donner une solution verte limpide. A cette solution, on ajoute 1,2 éq de CS₂ (1,42 ml, 1,2 mmol), et on agite pendant 30 minutes à 0°C pour obtenir une solution jaune orangée du sel de sodium du diphényldithiocarbamate.

On ajoute du malonate de bromodiéthyle (10 mmol) à la solution ci-dessus à -20°C et on amène lentement la température du mélange réactionnel jusqu'à la température ambiante. Après agitation pendant 2 heures à température ambiante, le mélange réactionnel est traité avec de l'eau et extrait avec de l'éther. La couche éthérée est séchée sur du MgSO₄ et concentrée.

Rendement : 51 %.

La pureté du produit a été vérifiée par RMN.

### 2. 1. 2. Synthèse du diphényl dithiocarbamate d'acétate de fluoroéthyle (DPFEA)

On place de l'hydrure de sodium (7 mmol/0.168 g, 1,3 eq dans 5 ml THF) dans un flacon séché à la flamme et on l'agite à 0°C. A ce mélange, on ajoute goutte à goutte de la diphénylamine (5,4 mmoles, 1 eq) dans 5 ml de THF, et 10 ml de DMSO et on agite pendant une heure. On ajoute du disulfure de carbone (2,3 eq) à la solution de 0°C et on l'agite pendant encore une demi-heure. On abaisse la température de la solution jusqu'à 18°C et on ajoute l'équivalent d'acétate de fluoroéthyle. Après addition, on élève lentement la température du mélange réactionnel jusqu'à la température ambiante et on l'agite pendant une demi-heure à température ambiante. Le produit obtenu est hydrolysé en ajoutant de l'eau et la couche organique est extraite avec de l'éther. L'extrait éthérée est concentré pour donner des cristaux jaunes de (DPFEA) et la pureté du produit est vérifiée par analyse RMN.

### 2. 2. Polymérisation

2. 2. 1. Le mode opératoire général pour la polymérisation est le suivant que l'agent de transfert de chaîne soit le diphényl dithiocarbamate de diéthylmalonate ou bien le dithiocarbamate d'acétate de fluoréthyle.

Dans ce qui suit, la vinylpyrrolidone est mentionnée, mais le procédé de préparation peut être généralisé à tout monomère éthylénique à cycle lactame.

### Première étape

### Préparation du macroamorceur polyvinylpyrrolidone

La polymérisation de la vinylpyrrolidone (VP) utilisant le diphényl dithiocarbamate de malonate de diéthyle en tant que réactif RAFT est réalisée en mettant en oeuvre l'AIBN en tant qu'amorceur.

Dans une expérience typique, la VP, le diphényl dithiocarbamate de malonate de diéthyle (rapport agent de transfert de chaîne/monomère = 1/100), l'AIBN (10 % de l'agent de transfert de chaîne) et du dioxane (rapport de 1/1 en volume par rapport au monomère) sont placés dans un tube Schlenk. Le mélange réactionnel est dégazé par trois cycles de congélation-mise sous vide-décongélation (« freeze-pump-thaw cycle »), puis fermé hermétiquement sous vide et chauffé dans un bain à température constante à 80°C.

Le macroamorceur « actif » que l'on peut représenter par 'X-PVP-X avec X= (Phenyl)₂NC(S)S- et X'=-CH(COOC₂H₅)₂ obtenu est purifié par précipitations répétées dans l'éther éthylique et est séché sous vide.

### Deuxième étape

### Polymérisation de la seconde séquence à l'extrémité du macroamorceur »actif »

La polymérisation du second monomère (qui est par exemple le méthacrylate de méthyle) a lieu en présence du macroamorceur PVP ci-dessus dans 1,5 ml de dioxane et en présence de AIBN (0,1 mol % par rapport au total du monomère et du macroamorceur).

Le mélange réactionnel est soumis à trois cycles de congélation-mise sous vide-décongélation et chauffé dans un bain d'huile sous agitation à 80°C pendant 20 heures.

Une fois la réaction terminée, le mélange réactionnel est dissous dans le dichlorométhane (quantité juste nécessaire à la dissolution) et il est précipité dans l'éther.

La solution éthérée trouble est filtrée et concentrée par évaporation instantanée « flash » et est ajoutée à du pentane ; le précipité obtenu est séché sous vide à environ 70°C pendant 8 heures.

2. 2. 2. En variante, la polymérisation peut être réalisée de la manière suivante : on effectue d'abord la synthèse d'un macroamorceur poly(méthacrylate de méthyle) par exemple, puis on effectue la polymérisation de la séquence de vinylpyrrolidone à l'extrémité de ce macroamorceur. On suit le même mode opératoire que dans le paragraphe 2. 2. 1.

### 2. 2. 3. Polymérisation en utilisant un xanthate comme agent de transfert de chaîne synthèse sans étape de purification intermédiaire (« one pot synthesis » en anglais)

De la N-vinylpyrrolidone (4 mol, 3,74 ; 10⁻² moles) (le mode opératoire peut s'appliquer aussi à un autre monomère éthylénique à cycle lactame), l'agent de transfert de chaîne (xanthate ci-dessus, 0,0839 g, rapport agent de transfert de chaîne/monomère=environ 1/100 en moles), et du dioxane (4 ml) sont placés dans un ballon préséché, et de l'AIBN (0,0061 g, 10 % en moles de l'agent de transfert de chaîne) y est ajouté sous azote.

Le mélange réactionnel est soumis à trois cycles de congélation-mise sous vide-décongélation et est chauffé dans un bain d'huile thermostaté à 80°C sous agitation. La réaction est arrêtée après 14 heures par refroidissement dans l'azote liquide.

Le second monomère, par exemple l'acrylate de butyle (3 ml), et du dioxane (3 ml) sont ajoutés à ce mélange réactionnel à température ambiante sous azote. Le mélange réactionnel est de nouveau soumis à trois cycles de congélation-mise sous vide-décongélation et est chauffé à 80°C pendant encore 24 heures.

Une fois la réaction terminée, le mélange réactionnel est dissous dans le dichlorométhane (quantité juste nécessaire à la dissolution) et il est précipité dans l'éther. La solution éthérée trouble est filtrée et concentrée par évaporation instantanée (« flash ») et elle est ajoutée à du pentane, le précipité obtenu est séché sous vide à environ 70°C pendant 8 heures.

### Caractérisations

- La conversion est mesurée par pesée du polymère.
- La masse du macroamorceur polyvinyllactame, par exemple PVP, est déterminée par chromatographie en phase gazeuse GPC (appareil Varian 9012®) avec des colonnes gels G4000 G3000 G2500 de TSK®, équipées d'un détecteur infrarouge VARIAN® RI 4. L'éluant est un mélange 80/20 eau méthanol contenant 0,1 M de nitrate de sodium. Le débit de l'éluant est de 0,5 ml/min. La calibration est effectuée par des étalons poly(oxyde d'éthylène) .

La mesure de masse n'est donc qu'une mesure comparative et les valeurs ne représentent pas les masses moléculaires réelles. Cependant, les résultats peuvent être utilisés pour noter la tendance dans l'évolution des masses moléculaires et également pour déterminer la dispersité en masse des chaînes.
- La masse du macroamorceur non hydrosoluble (polyméthacrylate de méthyle, par exemple) est déterminée par GPC (Varian® 9012) avec des colonnes gels G4000 G3000 G2500 de TSK®, équipées d'un détecteur infrarouge Varian® RI 4. L'éluant est le THF. L'étalon est le polystyrène.
- La masse globale (GPC) du copolymère est déterminée par chromatographie GPC en phase solvant (Varian® 9012) avec des colonnes gels G4000 G3000 G2500 de TSK®, équipées d'un détecteur infrarouge Varian® RI 4. L'éluant est le THF. L'étalon est le polystyrène.
- La proportion des différentes séquences est déterminée par RMN¹H (Bruker®, 200 MHz) en faisant le rapport des surfaces des pics correspondants aux monomères des différentes séquences (à savoir par exemple d'une part MMA et d'autre part VP). La masse globale (RMN) du copolymère est déduite avec ces valeurs et la masse du macroamorceur déterminée plus haut.

Après avoir décrit ci-dessus le mode opératoire général pour la synthèse des copolymères selon l'invention, on donne ci-après des exemples concernant la préparation de copolymères spécifiques conformes à l'invention (exemples 1, 2 et 3) ainsi qu'un exemple comparatif.

### Exemple comparatif

Dans cet exemple, on prépare un copolymère (non-conforme à l'invention) comprenant une séquence polyvinylpyrrolidone et une séquence poly(méthacrylate de méthyle) : (PVP) 80 %-b-(PMMA) 20 % selon le procédé 1 (« ATRP » inverse) décrit ci-dessus.

### Première étape

### Synthèse du macroamorceur polyvinylpyrrolidone-Br : PVP-Br

Du CuBr₂ et du Me₆TREN sont placés dans un ballon de 50 mL et on ajoute 5 mL de dioxane.

On ajoute ensuite de la vinylpyrrolidone suivie par de l'AIBN (azobisisobutyronitrile).

Le rapport dioxane/monomère est de 1/1 en volume.

Les proportions AIBN/CuBr₂/CuO/Me₆TREN sont : 1/1,5/0,15/3.

La réaction est réalisée à une température de 80°C pendant 3 heures 45. La conversion atteint 84 %.

### Deuxième étape

### Synthèse du copolymère : Polymérisation du méthacrylate de méthyle (MMA) à l'extrémité du macroamorceur polyvinylpyrrolidone (PVP-Br)

On ajoute le macroamorceur de PVP(PVP-Br) dans du CuBr (rapport 1 :1,5 par rapport au PVP-Br) et du Me₆TREN (1:3 par rapport au CuBr) dans le dioxane sous atmosphère d'azote.

Macroamorceur polyvinylpyrrolidone PVP-Br : 0,026 mM.

Méthacrylate de méthyle (MMA) : 3,87 mM.

La réaction est réalisée à une température de 100°C pendant 3 heures 45.

La conversion est de 60 %.

Les caractéristiques du macroamorceur et du copolymère final sont résumées dans le tableau suivant :

| Copolymère | VP/AIBN | Mn (macroamorceur PVP) (GPC) | % PVP/poids total | % PMMA/poids total | Mn PMMA déduit par RMN |
|---|---|---|---|---|---|
| (PVP) 80 %--b-- (PMMA) 20 % | 1/0,25 | 38 700 | 80,2 % | 19,8 % | 9600 |
| Vinylpyrrolidone = VP | | | | | |
| Azobisisobutyronitrile = AIBN | | | | | |
| Mₙ = masse moléculaire moyenne en nombre | | | | | |

Le polymère obtenu est dissous dans l'éthanol à raison de 6 % en poids.

Le polymère est étalé et donne un film friable. Il n'est pas possible d'obtenir un film continu.

### Exemple 1

Dans cet exemple, on prépare un copolymère (conforme à l'invention) comprenant une séquence de polyvinylpyrrolidone (PVP) et une séquence poly(acrylate de méthyle) (PAMe) : (PVP) 66 %-b-polyacrylate de méthyle (PAMe) 34 %, selon le procédé décrit dans le paragraphe 2.2.3. ci-dessus.

On place la N-vinylpyrrolidone (VP) (3,74 x 10⁻² moles soit 4,15 g), l'agent de transfert de chaîne (xanthate décrit plus haut, 0,0839 g, (rapport agent de transfert de chaîne/monomère : environ 1/100 en moles) et du dioxane (4 ml) dans un flacon séché au préalable ; et de l'AIBN (0,0061 g, 10 % en moles par rapport à l'agent de transfert de chaîne) est ajouté dans le flacon sous azote.

Ce mélange réactionnel est soumis à trois cycles congélation-mise sous vide-décongélation et il est chauffé dans un bain d'huile thermostaté à 80°C sous agitation.

La réaction est arrêtée après 14 heures par refroidissement dans l'azote liquide.

Le second monomère (méthacrylate de méthyle) (3 ml) et du dioxane (3 ml) sont ajoutés à ce mélange réactionnel à température ambiante sous azote. Le mélange réactionnel est de nouveau soumis à trois cycles congélation-mise sous vide-décongélation et est chauffé à 80°C pendant encore 24 heures.

Une fois la réaction terminée, le mélange réactionnel est dissous dans du dichlorométhane (quantité juste nécessaire pour la dissolution) et il est précipité dans l'éther.

La solution éthérée trouble est filtrée et concentrée par évaporation instantanée (« flash ») et elle est ajoutée à du pentane ; le précipité obtenu est séché sous vide à environ 70°C pendant huit heures, et il est caractérisé par GPC et RMN.

Les caractéristiques des réactifs et du procédé sont résumés ci-après.

Agent de transfert de chaîne : xanthate C₂H₅OC (S) SCH (CH₃) COOCH₃.

Rapport en moles xanthate/monomère VP : 1/100.

Rapport AIBN/agent de transfert de chaîne xanthate : 10 % en moles.

Rapport monomères/AIBN : 0,1.

Température de la réaction : 80°C.

Solvant : dioxane.

Les caractéristiques du macroamorceur et du copolymère final sont résumées dans le tableau suivant :

| Copolymère | Conversion VP | Mn (macroamorceur PVP) (GPC) | Conversion de l'acrylate | % de VP en poids (RMN) | Masses moléculaires du copolymère final (GPC) | | | |
|---|---|---|---|---|---|---|---|---|
| | | | | | Mp | Mn | Mw | PDI |
| PVP 66 % --b-- (PAMe) 34 % | 88 % | 12560 | 75 % | 34 | 42900 | 20500 | 44770 | 2,2 |
| Mn : masse moléculaire moyenne en nombre | | | | | | | | |
| Mw : masse moléculaire moyenne en poids | | | | | | | | |
| Mp : masse moléculaire au pic | | | | | | | | |
| PDI : Indice de polydispersité=Mw/Mn | | | | | | | | |
| Poly(acrylate de méthyle) : PAMe | | | | | | | | |

### Exemple 2

Dans cet exemple, on prépare un copolymère (conforme à l'invention) comprenant une séquence de polyvinylpyrrolidone (PVP) et une séquence poly(acrylate de butyle) (PABu) : (PVP) 60 %-b-(PABu) 40 %.

Le mode opératoire est le même que dans l'exemple 1 (procédé du paragraphe 2.2.3.), seules les proportions et la nature des monomères changent par rapport à l'exemple 1. Les caractéristiques du macroamorceur et du copolymère final sont résumées dans le tableau suivant :

| Copolymère | Conversion VP | Mn (macroamorceur PVP) (GPC) | % de VP en poids | Masses moléculaires du copolymère final (GPC) | | | |
|---|---|---|---|---|---|---|---|
| | | | | Mp | Mn | Mw | PDI |
| PVP 60 %--b--poly(acrylate de butyle) 40 % | 69 | 8520 | 60 | 55610 | 34000 | 66700 | 1,97 |

Le polymère est dissous dans l'éthanol à raison de 6% en poids.

Il donne un film non friable et non collant.

### Exemple 3

Dans cet exemple, on prépare un copolymère (conforme à l'invention) comprenant une séquence de polyvinylcaprolactame (PVCap) et une séquence de poly(acrylate de butyle) (PABu) :
(PVCap) 10 %-b-(PABu) 90%.

La synthèse du copolymère est réalisée selon le procédé du paragraphe 2.2.2.

### Première étape

Synthèse du macroamorceur (polyvinylcaprolactame « actif » (PVCap).

Du VCap (1 g, 7,183 mmoles), l'agent de transfert de chaîne DPFEA (0,0244 g ) (agent de transfert de chaîne = monomère NVCap/50 en moles) et l'AIBN (20 % en moles de l'agent de transfert de chaîne) dans le dioxane. sont placés dans un flacon séché au préalable et sont soumis à trois cycles de congélation-mise sous vide-décongélation pour éliminer l'oxygène présent. Le mélange réactionnel est immergé dans un bain d'huile thermostaté à 80°C et est agité pendant 17 heures.

La réaction est arrêtée par congélation dans l'azote liquide et une petite quantité de l'échantillon est prélevée à des fins d'analyse.

### Deuxième étape

### Synthèse du copolymère : Polymérisation de l'acrylate de butyle à l'extrémité du macroamorceur « actif » polyvinylcaprolactame (PVCap)

Le second monomère (acrylate de butyle, 1 ml) et le dioxane (1 ml) sont ajoutés directement au mélange réactionnel et l'oxygène est éliminé de nouveau par des cycles congélation-mise sous vide-décongélation. On poursuit la réaction pendant 24 heures à 60°C. Une fois que la réaction est terminée, le mélange réactionnel est dilué avec du dichlorométhane et il est précipité dans le pentane et séché sous vide à température ambiante.

Les caractéristiques du macroamorceur et du copolymère final sont résumées dans le tableau suivant :

| Copolymère | Mn (macroamorceur PVCap) (RMN) | % ABu dans le copolymère (RMN) | % ABu en poids dans le copolymère (RMN) | Mn PAbu (RMN) |
|---|---|---|---|---|
| PVCap-b-PMMA | 10400 | 88 % en moles | 88 | 68900 |

## Revendications

1. Copolymère éthylénique, séquencé, linéaire comprenant :
- au moins une séquence A susceptible d'être préparée à partir de monomères, comprenant de 52 à 100% en poids d'un monomère éthylénique à cycle lactame répondant à la formule (I) suivante : 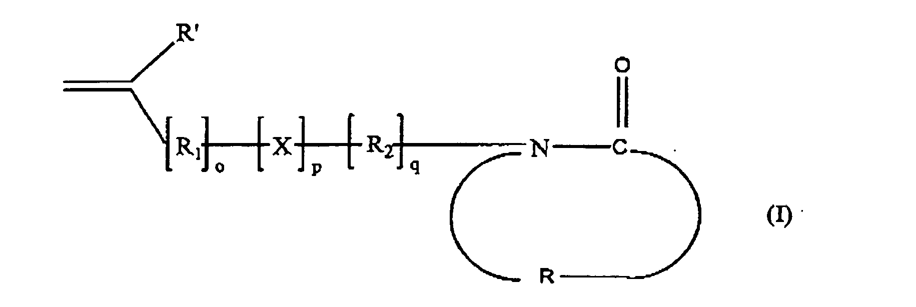 dans laquelle :
• R représente un groupe -(CH₂)ₙ-, où un ou plusieurs des atomes de carbone sont éventuellement remplacés par un atome d'azote ou un atome d'oxygène, où n est un nombre entier de 3 à 12, et où un ou plusieurs des atomes de carbone sont éventuellement substitués par un ou plusieurs groupes alkyle en C₁ à C₆ ;
• R' représente H ou un groupe méthyle ;
• R₁ et R₂, qui peuvent être identiques ou différents représentent un groupe alkylène ou aralkylène, linéaire, ramifié, ou cyclique, de 1 à 22 C, dans lequel un ou plusieurs des atomes de carbone sont éventuellement remplacés par un atome d'oxygène ou d'azote;
• X est choisi parmi -OCO-, -NHCO-, -COO-, -O- ;
• o, p, q, représentent chacun indépendamment les uns des autres 0 ou 1 ;
- et au moins une séquence B susceptible d'être préparée à partir de monomères ne comportant pas de monomère éthylénique à cycle lactame de formule (I) ou comportant une proportion minoritaire de celui-ci, ladite séquence B présentant une température de transition vitreuse Tg comprise entre -55°C à +55°C ; ladite séquence A représentant de 10 à 95% en poids du copolymère et ladite séquence B représentant de 5 à 90% en poids du poids total du copolymère.

2. Copolymère selon la revendication 1, dans lequel dans la formule (I), o est 0, p est 1, q est 1, R₂ représente -CH₂CH₂- ; X représente COO ou CONH, et R est (CH₂)₃ ou (CH₂)₅ ou CH₂CH₂NH.

3. Copolymère selon la revendication 1, dans lequel le monomère éthylénique à cycle lactame est un vinyllactame qui répond à la formule (II) suivante : 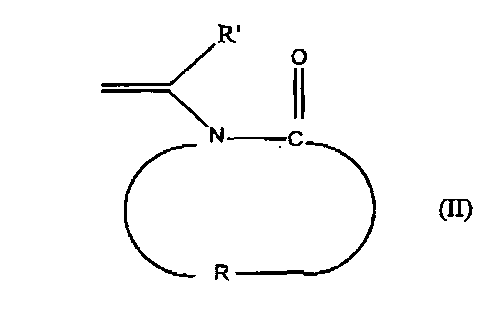 dans laquelle R et R' ont la signification déjà donnée dans la revendication 1.

4. Copolymère selon l'une quelconque des revendications 1 à 4, dans lequel dans les formules (I) et (II), R représente -(CH₂)ₙ-, où n est un nombre entier de 3 à 5, ou bien R représente -CH₂-CH₂-NH-.

5. Copolymère selon l'une quelconque des revendications précédentes, qui est un copolymère filmogène.

6. Copolymère selon l'une quelconque des revendications précédentes, qui a une masse moléculaire en nombre du copolymère global comprise entre 4 000 et 1 000 000, de préférence entre 4 000 et 800 000, de préférence encore entre 4 000 et 500 000.

7. Copolymère selon l'une quelconque des revendications précédentes, dans lequel la séquence A représente de 20 à 90 % en poids dupoids total du copolymère.

8. Copolymère selon l'une quelconque des revendications précédentes, dans lequel la séquence B représente 10 à 80 % en poids du poids total du copolymère.

9. Copolymère selon l'une quelconque des revendications précédentes, dans lequel la masse moléculaire en nombre de chaque séquence A ou B est comprise entre 2 000 et 1 000 000, de préférence encore comprise entre 2 000 et 800 000, et mieux comprise entre 2 000 et 500 000.

10. Copolymère selon l'une quelconque des revendications précédentes, dans lequel lesdits monomères à partir desquels est préparée ladite séquence A comprennent une proportion de monomère éthylénique à cycle lactame, par exemple de vinyllactame, de formule (I) ou (II), supérieure ou égale à 50% en poids par rapport au poids total des monomères de cette séquence A.

11. Copolymère selon la revendication 8, dans lequel le pourcentage de monomère éthylénique à cycle lactame, tel qu'un vinyllactame, de formule (I) ou (II), dans la séquence A est compris entre 55 et 95 % en poids, et est par exemple de 90 % en poids.

12. copolymère selon l'une quelconque des revendications précédentes, dans lequel ladite séquence A est un homopolymère du monomère éthylénique à cycle lactame, tel qu'un N-vinyllactame de formule (I) ou de formule (II).

13. Copolymère selon la revendication 1, dans lequel ledit N-vinyllactame de formule (I) est le pyrrilidinoéthylacrylate ou le pyrrilidinoéthylméthacrylate.

14. Copolymère selon la revendication 2, dans lequel ledit N-vinyllactame de formule (II) est le N-vinylpyrrolidone (n=3), le N-vinylpipéridinone (valérolactame) (n=4), le N-vinylcaprolactame (n=5), la N-vinylimidazolidinone dans laquelle R est le groupe -CH₂-CH₂-NH-, le N-vinyl-5-méthyl-2-pyrrolidone, le N-vinyl-5-éthyl-2-pyrrolidone, le N-vinyl-6-méthyl-2-pipéridone, le N-vinyl-6-éthyl-2-pipéridone, le N-vinyl-7-méthyl-2-caprolactame, ou le N-vinyl-7-éthyl-2-caprolactame.

15. Copolymère selon la revendication 14, dans lequel ladite séquence A est un homopolymère de N-vinylpyrrolidone.

16. Copolymère selon la revendication 14, dans lequel ladite séquence A est un homopolymère de N-vinylcaprolactame.

17. Copolymère selon la revendication 1, dans lequel la séquence A est un copolymère, susceptible d'être préparée à partir de monomères comprenant, outre le monomère éthylénique à cycle lactame, tel qu'un vinyllactame, de formule (I), un ou plusieurs autres monomères.

18. Copolymère selon la revendication 17, dans lequel la séquence A est un copolymère statistique, alterné ou à gradient.

19. Copolymère selon la revendication 17 ou 18, dans laquelle le ou lesdits autres monomères de la séquence A sont choisis parmi les monomères dont la température de transition vitreuse de l'homopolymère correspondant est inférieure ou égale à 50°C, de préférence inférieure ou égale à 20°C, mieux inférieure ou égale à 0 °C.

20. Copolymère selon la revendication 19, dans laquelle le ou lesdits autres monomères de la séquence A ont en outre une température de transition vitreuse de l'homopolymère correspondant supérieure ou égale à - 150°C.

21. Copolymère selon la revendication 19, dans lequel le ou lesdits autres monomères de la séquence A dont la température de transition vitreuse de l'homopolymère correspondant est inférieure ou égale à 50°C est(sont) choisi(s) parmi : l'acrylate de t-butyle (Tg=50°C), l'acétate de vinyle (Tg=23°C), le méthacrylate de butyle (Tg=20°C), l'acrylate de cyclohexyle (19°C), l'acrylate d'hydroxyéthyle (15°C), l'acrylate de méthyle (Tg=10°C), le méthacrylate d'éthoxyéthyle (Tg=0°C), le méthacrylate de n-hexyle (-5°C), l'acrylate d'éthyle (Tg=-24°C), l'acrylate d'isobutyle (-24°C), le vinylméthyle ether (-34°C), l'acrylate de méthoxyéthyle (-33°C), l'acrylate de n-butyle (Tg=-54°C), l'acrylate d'éthylhexyle (Tg=-50°C), le méthacrylate de POE (n=8 à 10) (Tg=-55°C), et le méthacrylate d'isobornyle ; le butylacrylamide, le N-isopropylacrylamide, le N,N-diméthylacrylamide et le N,N-dibutylacrylamide, l'acrylate d'hexyle, l'acrylate d'octyle, l'acrylate de lauryle, l'acrylate d'isooctyle, le méthacrylate d'éthylhéxyle, le méthacrylate d'octyle,le méthacrylate de lauryle, le néononanoate de vinyle, le néododécanoate de vinyle ; et le N-octylacrylamide.

22. Copolymère selon la revendication 17, dans lequel un ou plusieurs parmi lesdits autres monomères de la séquence A sont choisis parmi les monomères hydrophiles.

23. Copolymère selon la revendication 17, dans lequel un ou plusieurs parmi lesdits autres monomères de la séquence A sont choisis parmi les monomères non hydrophiles.

24. Copolymère selon l'une quelconque des revendications précédentes, dans lequel la séquence A, a une température de transition vitreuse globale de la séquence de 0 à 250°C, de préférence de 0 à 220°C et de préférence encore de 5 à 200°C.

25. Copolymère selon l'une quelconque des revendications précédentes, dans lequel la séquence A est une séquence hydrophile.

26. Copolymère selon l'une quelconque des revendications précédentes, dans lequel la ou les séquences B autres que la séquence A est(sont) susceptible(s) d'être préparée(s) à partir du ou de plusieurs monomères éthyléniques choisis parmi : les monomères allyliques les acrylates, les méthacrylates, les acrylamides, les méthacrylamides, les monomères vinyliques, et leurs mélanges, et éventuellement des monomères éthyléniques à cycle lactame, par exemple des monomères de vinyllactame, de formule (I) ou (II), la proportion en poids desdits monomères (I) ou (II) étant inférieure à 50 % en poids, de préférence inférieure ou égale à 40 % et, de préférence encore inférieure ou égale à 30% en poids.

27. Copolymère selon l'une quelconque des revendications précédentes, dans lequel la séquence B a une température de transition vitreuse Tg pouvant varier dans l'intervalle allant de -50°C à +50°C, de préférence de -50°C à +45°C.

28. Copolymère selon l'une quelconque des revendications précédentes, dans lequel le ou les monomère(s) constituant la séquence B ont une longueur de chaîne carbonée inférieure ou égale à 10 atomes de carbone.

29. Copolymère selon l'une quelconque des revendications précédentes, dans lequel le ou les monomère(s) constituant(s) la séquence B ont une longueur de chaîne carbonée supérieure ou égale à 12 atomes de carbone et la proportion de ces monomères dans la séquence B est inférieure à 50 % en poids.

30. Copolymère selon l'une quelconque des revendications précédentes, dont la séquence B, de température de transition vitreuse Tg comprise entre -55°C et +55°C, qui est un homopolymère ou un copolymère, est issue de un ou plusieurs monomères, qui sont tels que les homopolymères préparés à partir de ces monomères peuvent présenter des températures de transition vitreuse variant de -200°C à +250°C.

31. Copolymère selon la revendication 30, dans lequel la séquence B est formée d'un copolymère. constitué à partir d'un premier monomère dont la Tg de l'homopolymère correspondant est dans la plage de +20°C à +250°C, tel que le méthacrylate de méthyle (MMA) ou le styrène, et d'un second monomère dont la Tg de l'homopolymère correspondant est dans la plage de -200°C à +20°C.

32. Copolymère selon la revendication 30, dans lequel la séquence B dont la température de transition vitreuse est comprise entre -55°C et +55°C est un homopolymère constitué par un seul type de monomère dont la Tg de l'homopolymère correspondant est dans la plage allant de -55°C à +55°C, de préférence de -50°C à +50°C et de préférence de -50°C à +45°C.

33. Copolymère selon l'une quelconque des revendications précédentes, dans lequel le ou les monomères à partir duquel ou desquels la ou les séquence(s) B est(sont) susceptible(s) d'être préparée(s) est (sont) choisi(s) parmi les monomères suivants :
- Les hydrocarbures éthyléniques de 2 à 10 C, tels que l'éthylène, l'isoprène, ou le butadiène ;
- Les acrylates de formule CH₂ = CHCOOR₃ ;
- Les méthacrylates de formule : 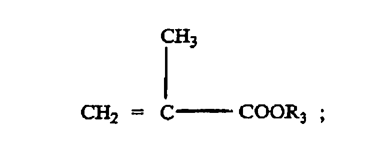 dans lesquelles R₃ représente :
• un groupe alkyle linéaire, ou ramifié, de 1 à 18 atomes de carbone, dans lequel se trouve(nt) éventuellement intercalé(s) un ou plusieurs hétéroatomes choisis parmi 0, N, S et P,
ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅) , où R₄ et R₅ identiques ou différents représentent un groupe alkyle en C₁ à C₆ ou un groupe phényle,
des exemples de ces groupes alkyle sont méthyle, éthyle, propyle, butyle, isobutyle, tertiobutyle, éthylhexyle, octyle, lauryle, stéaryle,
des exemples de ces groupes dérivés d'alkyle sont les groupes hydroxyalkyle en C₁₋₄ tels que 2-hydroxyéthyle et 2-hydroxypropyle, et les groupes alcoxy (C₁₋₄) alkyle (C₁₋₄) tel que méthoxyéthyle, éthoxyéthyle et méthoxypropyle,
• un groupe cycloalkyle en C₃ à C₁₂, tel que le groupe isobornyle,
• un groupe aryle en C₃ à C₂₀ tel que le groupe phényle,
• un groupe aralkyle en C₄ à C₃₀ (groupe alkyle en C₁ à C₈) tel que 2- phényl-éthyle ou benzyle,
• un groupe hétérocyclique de 4 à 12 chaînons contenant un ou plusieurs hétéroatomes choisis parmi O, N, et S, le cycle étant aromatique ou non,
• un groupe hétérocyclylalkyle (alkyle de 1 à 4 C), tel que furfurylméthyle ou tétrahydrofurfurylméthyle, lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle, les atomes d'halogène (Cl, Br, I et F) , et les groupes Si (R₄R₅) , où R₄ et R₅ identiques ou différents représentent un groupe alkyle en C₁ à C₆, ou un groupe phényle,
des exemples de groupes R₃ sont les groupes méthyle, éthyle, propyle, isobutyle, n-butyle, tertiobutyle isobutyle, héxyle, éthylhéxyle, octyle, lauryle, isooctyle, isodécyle, dodécyle, cyclohexyle, t-butylcyclohexyle, t-butylbenzyle, d'isobornyle, phényle, furfuryléméthyle, tétrahydrofurfurylmethyle, 2-hydroxyéthyle, 2-hydroxypropyle, 2-hydroxybutyle, méthoxyéthyle, éthoxyéthyle, méthoxyéthyle, méthoxypropyle, éthyl-2-perfluorohexyle,
• un autre exemple de groupe R₃ sont les groupes R₃=-(OC₂H₄)ₘ-OR", avec m = 5 à 150 et, R" = H ou alkyle de C₁ à C₃₀, par exemple -POE- méthoxy ; -POE-béhényle ;
- Les (méth)acrylamides de formule : 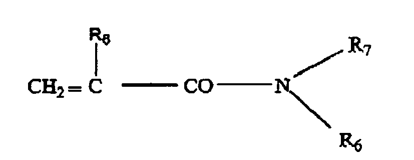 où
R₈ désigne H ou méthyle ;
et R₇ et R₆ identiques ou différents représentent chacun un atome d'hydrogène ou un groupe alkyle de 1 à 18 atomes de carbone linéaire ou ramifié, dans lequel se trouve(nt), éventuellement intercalé(s) un ou plusieurs hétéroatomes choisis parmi 0, N, S et P, ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅), où R₄ et R₅ identiques ou différents, représentent un groupe alkyle en C₁ à C₆, ou un groupe phényle, des exemples de ces groupes sont les groupes méthyle, éthyle, n-butyle, t-butyle, isopropyle, isohexyle, isooctyle, isononyle, et hydroxyalkyle en C₁₋₄ tels que 2-hydroxypropyle,
• un groupe cycloalkyle en C₃ à C₁₂, tel que le groupe isobornyle,
• un groupe aryle en C₃ à C₂₀ tel que phényle,
• un groupe aralkyle en C₄ à C₃₀ (groupe alkyle en C₁ à C₉) tel que 2- phényl éthyle ou benzyle,
• un groupe hétérocyclique de 4 à 12 chaînons contenant un ou plusieurs hétéroatomes choisis parmi O, N, et S, le cycle étant aromatique ou non,
• un groupe hétérocyclylalkyle (alkyle de 1 à 4 C), tel que furfurylméthyle ou tétrahydrofurfurylméthyle, lesdits groupes cycloalkyle, aryle, aralkyle, hétérocyclique ou hétérocyclylalkyle pouvant être éventuellement substitué(s) par un ou plusieurs substituants choisi(s) parmi les groupes hydroxyle, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si (R₄R₅) , où R₄ et R₅ identiques ou différents représentent un groupe alkyle en C₁ à C₆, ou un groupe phényle, des exemples de monomères (méth)acrylamide sont le (méth)acrylamide, le N-éthyl-(méth)acrylamide, le N-butylacrylamide, le N-t-butylacrylamide, le N-isopropylacrylamide, le N,N-diméthyl(méth)acrylamide, le N,N-dibutylacrylamide, le N-octylacrylamide, le N-dodécylcacrylamide, l'undécylacrylamide, et le N (2-hydroxypropylméthacrylamide);
- Les composés allyliques de formule :
CH₂ = CH-CH₂-R₉ ou CH₂ = C (CH₃) -CH₂-R₉ ;
- Les composés vinyliques de formule :
CH₂ = CH-R₉,
où R₉ est un groupe
- hydroxyle,
- Cl,
- NH₂,
- OR₁₀, où R₁₀ représente un groupe phényle ou un groupe alkyle en C₁ à C₁₂ (le monomère est un éther de vinyle ou d'allyle),
- Acétamide : NHCOCH₃,
- OCOR₁₁, où R₁₁ représente :
• un groupe alkyle de 2 à 12 C linéaire ou ramifié (le monomère est un ester de vinyle ou d'allyle) ;
• un groupe cycloalkyle en C₃ à C₁₂ tel que isobornyle, cyclohexyle,
• un groupe aryle en C₃ à C₂₀ tel que phényle,
• un groupe aralkyle en C₄ à C₃₀ (groupe alkyle en C₁ à C₈) tel que 2-phényléthyle ; benzyle,
• un groupe hétérocyclique de 4 à 12 chaînons contenant un ou plusieurs hétéroatomes choisis parmi O, N, et S, le cycle étant aromatique ou non,
• un groupe hétérocyclylalkyle (alkyle de 1 à 4 C), tel que furfurylméthyle ou tétrahydrofurfurylméthyle,
lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve (nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si(R₄R₅) où R₄ et R₅ identiques ou différents représentent un groupe alkyle en C₁ à C₆, ou un groupe phényle,
des exemples de monomères vinyliques sont le vinylcyclohexane, et le styrène,
des exemples d'esters de vinyle sont : l'acétate de vinyle le propionate de vinyle, le butyrate de vinyle, l'éthylhexanoate de vinyle, le néononanoate de vinyle ; et le néododécanoate de vinyle,
parmi les éthers de vinyle on trouve par exemple le vinyl méthyl ether, le vinyl éthyl éther, et le vinyl isobutyl éther ;
- Les monomères (méth)acryliques ou (méth)acrylamides ou vinyliques à groupe fluoré ou perfluoré, tels que le méthacrylate d'éthyl perfluorooctyle ;
- Les monomères (méth)acryliques ou vinyliques siliconés, tels que le méthacryloxypropyltris(triméthylsiloxy)silane, l'acryloxypropylpolydiméthylsiloxane, ou les (méth)acrylamides siliconés.

34. Copolymère selon la revendication 33, dans lequel le ou les monomère(s), à partir duquel ou desquels le ou les séquence(s) B est (sont) susceptible(s) d'être préparée(s) est(sont) choisi(s) parmi les monomères suivants dont la température de transition vitreuse de l'homopolymère correspondant se situe dans la plage de -55°C à +55°C, tels que :
le méthacrylate d'hydroxyéthyle (55°C), le méthacrylate d'isobutyle(53°C),l'acrylate de n-hexyle (Tg=45°C), l'acrylate de t-butyle (Tg=50°C) , l'acétate de vinyle (Tg=23°C), le méthacrylate de butyle (Tg=20°C), l'acrylate de cyclohexyle (19°C), l'acrylate d' hydroxyéthyle (15°C), l'acrylate de méthyle (Tg=10°C),le méthacrylate d'éthoxyéthyle (Tg=0°C), le méthacrylate de n-hexyle (-5°C), ), le butyrate de vinyle (-5°C), l'acrylate d'éthyle (Tg=-24°C), l'acrylate d'isobutyle (-24°C), le vinylméthyle ether (-34°C), l'acrylate de méthoxyéthyle (-33°C), l'acrylate de n-hexyle (-45°C), l'acrylate de n-butyle (Tg=-54°C), l'acrylate d'éthylhexyle (Tg=-50°C), le méthacrylate de POE (n=8 à 10) (Tg=-55°C) ; et parmi d'autres monomères dont la Tg est en dehors de la plage de -55°C à +55°C, tels que les monomères suivants : acrylate de t-butylcyclohexyle, acrylate de t-butylbenzyle, acrylate de furfuryle et acrylate d'isobornyle ; méthacrylate de méthyle, méthacrylate d'éthyle, méthacrylate de cyclohexyle, méthacrylate de t-butylcyclohexyle, méthacrylate de t-butylbenzyle, et méthacrylate d'isobornyle ;
N-butylacrylamide, N-t-butylacrylamide, N-isopropylacrylamide, N,N-diméthylacrylamide et N,N-dibutylacrylamide ;
vinylcyclohexane, styrène ;
acrylate d'hexyle, acrylate d'octyle,
acrylate de lauryle, acrylate d'isooctyle, acrylate d'isodécyle ;
méthacrylate d'éthylhéxyle, méthacrylate d'octyle, méthacrylate de lauryle, méthacrylate d'isooctyle, méthacrylate d'isodécyle ;
néononanoate de vinyle, le néododécanoate de vinyle ;
N-octylacrylamide.

35. Copolymère selon l'une quelconque des revendications 26 à 34, dans lequel un ou plusieurs parmi les monomères constituant la ou les séquences B sont choisis parmi les monomères hydrophiles.

36. Copolymère selon l'une quelconque des revendications précédentes, dans lequel une ou plusieurs parmi les séquences B autres que la séquence A sont hydrophiles.

37. Copolymère selon la revendication 22 ou la revendication 35, dans lequel les monomères hydrophiles sont choisis parmi les monomères cationiques, les monomères anioniques et les monomères non ioniques.

38. Copolymère selon la revendication 23, dans lequel le ou les monomères non hydrophiles de la séquence A sont choisis parmi les monomères constituant la séquence B, tels que définis dans l'une quelconque des revendications 25 à 33 en respectant les critères de Tg et d'hydrophilie souhaités.

39. Copolymère selon la revendication 37, dans lequel les monomères cationiques sont choisis parmi la 2-vinylpyridine; la 4-vinylpyridine, le (méth)acrylate de diméthylaminoéthyle ; le (méth)acrylate de diéthylaminoéthyle ; le (méth)acrylamide de diméthylaminopropyle ; et les formes salifiées ou quaternisées de ceux-ci.

40. Copolymère selon la revendication 39, dans lequel les monomères anioniques sont choisis parmi l'acide acrylique, l'acide méthacrylique, l'acide crotonique, l'anhydride maléique, l'acide itaconique, l'acide fumarique, l'acide maléique, l'acide styrènesulfonique, l'acide 2-acrylamido 2-méthylpropanesulfonique, l'acide vinylbenzoïque, l'acide vinylphosphonique, le (méth)acrylate de sulfopropyle et les sels de ceux-ci.

41. Copolymère selon la revendication 39, dans lequel les monomères non-ioniques sont choisis parmi :
- les carboxybétaïnes ou sulfobétaines éthyléniques obtenues, par exemple par quaternisation de monomères à insaturation éthylénique comportant une fonction amine par des sels d'acide carboxylique à halogène mobile, par exemple, le chloroacétate de sodium, ou par des sulfones cycliques, par exemple la propanesulfone ;
- les (méth)acrylates ou (métha)crylamides d'hydroxyalkyle dont le groupe alkyle a de 2 à 4 atomes de C, en particulier le (méth)acrylate d'hydroxyéthyle
ou d'hydroxypropyle,
- les (méth)acrylates ou (métha)crylamides d' alcoxy (C₁₋₄) alkyle (C₁₋₄) tel que méthoxyéthyle, éthoxyéthyle et méthoxypropyle,
- les (méth)acrylates ou (métha)crylamides à groupe - (OC₂H₄)ₘ-OR", avec m = 5 à 150 et , R" = H ou alkyle de C₁ à C₄, par exemple -POE- méthoxy -POE-OH ;
- les vinyllactames ;
- et les (méth)acrylates de polysaccharide comme l'acrylate de saccharose.

42. Copolymère selon l'une quelconque des revendications précédentes, choisi parmi les copolymères biséquencés de type AB, les copolymères triséquencés de type ABA, BAB, ABC, ACB, c'est-à-dire les copolymères comportant outre les séquences A et B une autre séquence C différente des séquences A et B, les copolymères multiséquencés ayant plus de trois séquences de type (AB)ₙ, (ABA)ₙ, (BAB)ₙ, (ABC)ₙ, avec C différent de A ou B, et les copolymères multiséquencés ayant plus de trois séquences différentes de type ABCD.

43. Composition cosmétique comprenant un copolymère éthylénique, séquencé, linéaire comprenant :
- au moins une séquence A susceptible d'être préparée à partir de monomères, comprenant de 52 à 100% en poids d'un monomère éthylénique à cycle lactame répondant à la formule (I) suivante : 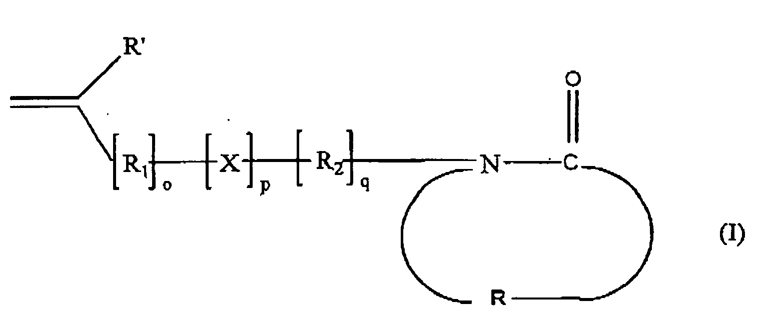 dans laquelle :
• R représente un groupe - (CH₂)ₙ-, où un ou plusieurs des atomes de carbone sont éventuellement remplacés par un atome d'azote ou un atome d'oxygène, où n est un nombre entier de 3 à 12, et où un ou plusieurs des atomes de carbone sont éventuellement substitués par un ou plusieurs groupes alkyle en C₁ à C₆ ;
• R' représente H ou un groupe méthyle ;
• R₁ et R₂, qui peuvent être identiques ou différents représentent un groupe alkylène ou aralkylène, linéaire, ramifié, ou cyclique, de 1 à 22 C, dans lequel un ou plusieurs des atomes de carbone sont éventuellement remplacés par un atome d'oxygène ou d'azote ;
• X est choisi parmi -OCO-, -NHCO-, -COO-, -O- ;
• o, p, q, représentent chacun indépendamment les uns des autres 0 ou 1 ;
- et au moins une séquence B susceptible d'être préparée à partir de monomères ne comportant pas de monomère éthylénique à cycle lactame de formule (I) ou comportant une proportion minoritaire de celui-ci, ladite séquence B présentant une température de transition vitreuse Tg comprise entre -55°C à +55°C ; ladite séquence A et ladite séquence B représentant de 1 à 99% en poids du poids total du copolymère.

44. Composition cosmétique selon la revendication 43, contenant de 0,1 à 60 % en poids, de préférence de 0,5 % à 50 % en poids, et de préférence encore de 1 à 40 % en poids du copolymère.

45. Composition selon l'une quelconque des revendications 43 et 44, comprenant outre ledit copolymère un milieu physiologiquement acceptable, dans lequel le copolymère se trouve sous forme dissoute ou dispersée.

46. Composition selon l'une quelconque des revendications 43 à 45, dans laquelle le milieu physiologiquement acceptable comprend un ou plusieurs solvants appropriés formant une phase hydrophile choisis parmi l'eau et les mélanges d'eau et de solvant (s) organique (s) hydrophile(s), tels que les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols.

47. Composition selon la revendication 46, dans laquelle la phase hydrophile contient, en outre, des éthers en C₁ à C₂.

48. Composition cosmétique selon l'une quelconque des revendications 43 à 47, dans laquelle ledit milieu physiologiquement acceptable comprend, en outre, une phase grasse composée de corps gras liquides ou solides à température ambiante, d'origine animale, végétale, minérale ou synthétique.

49. Composition selon l'une quelconque des revendications 43 à 48, comprenant, en outre, un ou plusieurs solvants organiques cosmétiquement acceptables.

50. Composition cosmétique selon l'une quelconque des revendications 43 à 49, dans laquelle ledit milieu physiologiquement acceptable comprend, en outre, un ou plusieurs agents auxiliaires de filmification choisis parmi les agents plastifiants et les agents de coalescence.

51. Composition cosmétique selon l'une quelconque des revendications 43 à 50, comprenant, en outre, une ou des matières colorantes choisies parmi les colorants hydrosolubles, et les matières colorantes pulvérulentes, comme les pigments, les nacres et les paillettes.

52. Composition selon l'une quelconque des revendications 43 à 51, comprenant, en outre, des charges.

53. Composition cosmétique selon l'une quelconque des revendications 43 à 52, comprenant, en outre, un ou plusieurs ingrédient(s) couramment utilisé (s) en cosmétique, tels que les vitamines, les épaississants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents antipelliculaires, les agents propulseurs, les polymères hydrosolubles, liposolubles ou en dispersion dans l'eau ou dans une phase grasse, filmogènes ou non, ou leurs mélanges.

54. Composition cosmétique selon l'une quelconque des revendications 43 à 53, **caractérisée par le fait qu'**elle se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple ou de pâte anhydre.

55. Composition cosmétique selon l'une quelconque des revendications 43 à 54, **caractérisée par le fait qu'**il s'agit d'un produit capillaire, tel qu'une laque ou un shampooing.

56. Composition cosmétique selon l'une quelconque des revendications 43 à 55, **caractérisée par le fait qu'**il s'agit d'une composition de maquillage, telle qu'un vernis à ongles.

57. Procédé cosmétique de maquillage ou de soin des matières kératiniques comprenant l'application sur les matières kératiniques d'une composition selon l'une des revendications 43 à 56.

58. Utilisation du copolymère tel que défini dans la revendication 43, pour améliorer la tenue de la coiffure, sans collant, d'une laque pour cheveux.

59. Utilisation du copolymère tel que défini dans la revendication 43, pour améliorer l'adhérence et la résistance à l'usure, sans collant, d'un vernis à ongles.

60. Utilisation du copolymère tel que défini dans la revendication 43, dans une composition cosmétique, telle qu'une composition de maquillage, pour masquer les rides et donner à la peau un aspect lissé, sans tiraillement.

## Claims

1. Linear block ethylenic copolymer comprising:
- at least one block A that may be prepared from monomers comprising from 52 to 100% by weight of an ethylenic monomer containing a lactam ring, corresponding to formula (I) below: 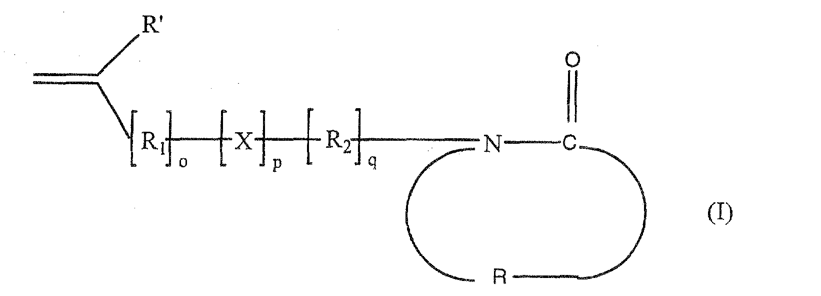 in which:
• R represents a group -(CH₂)ₙ-, in which one or more of the carbon atoms are optionally replaced with a nitrogen atom or an oxygen atom, in which n is an integer from 3 to 12, and in which one or more of the carbon atoms are optionally substituted with one or more C₁ to C₆ alkyl groups;
• R' represents H or a methyl group;
• R₁ and R₂, which may be identical or different, represent a linear, branched or cyclic alkylene or aralkylene group of 1 to 22 C, in which one or more of the carbon atoms are optionally replaced with an oxygen or nitrogen atom;
· X is chosen from -OCO-, -NHCO-, -COO- and -O-:
· o, p and q represent, independently of each other, 0 or 1;
- and at least one block B that may be prepared from monomers not comprising an ethylenic monomer containing a lactam ring of formula (I), or comprising a minor proportion thereof, the said block B having a glass transition temperature Tg of between -55°C and +55°C, said block A representing 10 to 95% by weight of the copolymer and said block B representing 5 to 90% by weight of the copolymer.

2. Copolymer according to claim 1, in which, in formula 9I), o is 0, p is 1, q is 1, R₂ represents -CH₂CH₂-; X represents COO or CONH, and R is (CH₂)₃, (CH2)5 or CH₂CH₂NH.

3. Copolymer according to claim 1, in which the ethylenic monomer containing a lactam ring is a vinyllactam that corresponds to formula (II) below: 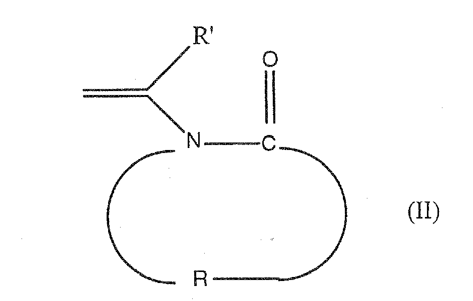 in which R and R' have the meaning already given in claim 1.

4. Copolymer according to any one of claims 1 to 4, in which, in formulae (I) and (II), R represents -(CHz)n- in which n is an integer from 3 to 5, or R represents -CH₂-CH₂-NH-.

5. Copolymer according to any one of the preceding claims, which is a film-forming copolymer.

6. Copolymer according to any one of the preceding claims, which has a number-average molecular mass of the global copolymer of between 4,000 and 1,000,000, preferably between 4,000 and 800,000 and more preferably between 4,000 and 500,000.

7. Copolymer according to any one of the preceding claims, in which the block A represents from 20% to 90% by weight of the total weight of the copolymer.

8. Copolymer according to any one of the preceding claims, in which the block B represents from 10% to 80% by weight of the total weight of the copolymer.

9. Copolymer according to any one of the preceding claims, in which the number-average molecular mass of each block A or B is between 2,000 and 1,000,000, preferably between 2,000 and 800,000 and better still between 2,000 and 500,000.

10. Copolymer according to any one of the preceding claims, in which the said monomers from which the said block A is prepared comprise a proportion of ethylenic monomer containing a lactam ring, for example of vinyllactam, of formula (I) or (II), of greater than or equal to 50% by weight relative to the total weight of the monomers of this block A.

11. Copolymer according to Claim 8, in which the percentage of ethylenic monomer containing a lactam ring, such as a vinyllactam, of formula (I) or (II) in the block A is between 55% and 95% by weight, for example 90% by weight.

12. Copolymer according to any one of the preceding claims, in which the said block A is a homopolymer of the ethylenic monomer containing a lactam ring, such as an N-vinyllactam of formula (I) or of formula (II).

13. Copolymer according to Claim 1, in which the said N-vinyllactam of formula (I) is pyrrilidinoethyl acrylate or pyrrilidinoethyl methacrylate.

14. Copolymer according to Claim 2, in which the said N-vinyllactam of formula (II) is N-vinylpyrrolidone (n=3), N-vinylpiperidinone (valerolactam) (n=4), N-vinylcaprolactam (n=5), N-vinylimidazolidinone in which R is a -CH₂-CH₂-NH- group, N-vinyl-5-methyl-2-pyrrolidone, N-vinyl-5-ethyl-2-pyrrolidone, N-vinyl-6-methyl-2-piperidone, N-vinyl-6-ethyl-2-piperidone, N-vinyl-7-methyl-2-caprolactam or N-vinyl-7-ethyl-2-caprolactam.

15. Copolymer according to Claim 14, in which the said block A is an N-vinylpyrrolidone homopolymer.

16. Copolymer according to Claim 14, in which the said block A is an N-vinylcaprolactam homopolymer.

17. Copolymer according to Claim 1, in which the block A is a copolymer that may be prepared from monomers comprising, besides the ethylenic monomer containing a lactam ring, such as a vinyllactam, of formula (I), one or more other monomers.

18. Copolymer according to Claim 17, in which the block A is a random, alternating or gradient copolymer.

19. Copolymer according to Claim 17 or 18, in which the said other monomer(s) of the block A is(are) chosen from monomers for which the glass transition temperature of the corresponding homopolymer is less than or equal to 50°C, preferably less than or equal to 20°C and better still less than or equal to 0°C.

20. Copolymer according to Claim 19, in which the said other monomer(s) of the block A also have a glass transition temperature for the corresponding homopolymer of greater than or equal to -150°C.

21. Copolymer according to Claim 19, in which the said other monomer(s) of the block A for which the glass transition temperature of the corresponding homopolymer is less than or equal to 50°C is(are) chosen from: t-butyl acrylate (Tg = 50°C), vinyl acetate (Tg = 23°C), butyl methacrylate (Tg = 20°C), cyclohexyl acrylate (Tg = 19°C), hydroxyethyl acrylate (Tg = 15°C), methyl acrylate (Tg = 10°C), ethoxyethyl methacrylate (Tg = 0°C), n-hexyl methacrylate (Tg = -5°C), ethyl acrylate (Tg = -24°C), isobutyl acrylate (Tg = -24°C), methyl vinyl ether (Tg = -34°C), methoxyethyl acrylate (Tg = -33°C), n-butyl acrylate (Tg = -54°C), ethylhexyl acrylate (Tg = -50°C), POE methacrylate (n=8 to 10) (Tg = -55°C) and isobornyl methacrylate; butylacrylamide, N-isopropylacrylamide, N,N-dimethylacrylamide and N,N-dibutylacrylamide, hexyl acrylate, octyl acrylate, lauryl acrylate, isooctyl acrylate, ethylhexyl methacrylate, octyl methacrylate, lauryl methacrylate, vinyl neononanoate, vinyl neododecanoate; and N-octylacrylamide.

22. Copolymer according to Claim 17, in which one or more of the said other monomers of the block A are chosen from hydrophilic monomers.

23. Copolymer according to Claim 17, in which one or more of the said other monomers of the block A are chosen from non-hydrophilic monomers.

24. Copolymer according to any one of the preceding claims, in which the block A, has an overall glass transition temperature for the block of from 0 to 250°C, preferably from 0 to 220°C and more preferably from 5 to 200°C.

25. Copolymer according to any one of the preceding claims, in which the block A is a hydrophilic block.

26. Copolymer according to any one of the preceding claims, in which the block(s) B other than the block A may be prepared from one or more ethylenic monomers chosen from: allylic monomers, acrylates, methacrylates, acrylamides, methacrylamides and vinyl monomers, and mixtures thereof, and optionally ethylenic monomers containing a lactam ring, such as vinyllactam monomers, of formula (I) or (II), the weight proportion of the said monomers (I) or (II) being less than 50% by weight, preferably less than or equal to 40% by weight and more preferably less than or equal to 30% by weight.

27. Copolymer according to any one of the preceding claims, in which the block B has a glass transition temperature Tg that may vary in the range from -50°C to +50°C and preferably from -50°C to +45°C.

28. Copolymer according to any one of the preceding claims, in which the monomer(s) constituting the block B has(have) a carbon chain length of less than or equal to 10 carbon atoms.

29. Copolymer according to any one of the preceding claims, in which the monomer(s) constituting the block B has(have) a carbon chain length of greater than or equal to 12 carbon atoms and the proportion of these monomers in the block B is less than 50% by weight.

30. Copolymer according to any one of the preceding claims, for which the block B, with a glass transition temperature Tg of between -55°C and +55°C, which is a homopolymer or a copolymer, is derived from one or more monomers, which are such that the homopolymers prepared from these monomers may have glass transition temperatures ranging from -200°C to +250°C.

31. Copolymer according to Claim 30, in which the block B is formed from a copolymer consisting of a first monomer for which the Tg of the corresponding homopolymer is in the range from +20°C to +250°C, such as methyl methacrylate (MMA) or styrene, and of a second monomer for which the Tg of the corresponding homopolymer is in the range from -200°C to +20°C.

32. Copolymer according to Claim 30, in which the block B whose glass transition temperature is between -55°C and +55°C is a homopolymer consisting of only one type of monomer for which the Tg of the corresponding homopolymer is in the range from -55°C to +55°C, preferably from -50°C to +50°C and preferentially from -50°C to +45°C.

33. Copolymer according to any one of the preceding claims, in which the monomer(s) from which the block(s) B may be prepared is(are) chosen from the following monomers:
- ethylenic hydrocarbons of 2 to 10 C, such as ethylene, isoprene or butadiene;
- the acrylates of formula CH₂ = CHCOOR₃;
- the methacrylates of formula: 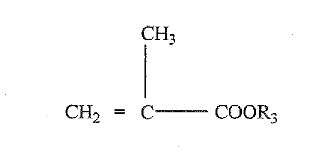 in which R₃ represents:
• a linear or branched alkyl group of 1 to 18 carbon atoms in which one or more hetero atoms chosen from 0, N, S and P is (are) optionally intercalated,
the said alkyl group also possibly being optionally substituted with one or more substituents chosen from hydroxyl groups, halogen atoms (Cl, Br, I and F) and groups Si(R₄R₅), in which R₄ and R₅, which may be identical or different, represent a C₁ to C₆ alkyl group or a phenyl group,
examples of these alkyl groups are methyl, ethyl, propyl, butyl, isobutyl, tert-butyl, ethylhexyl, octyl, lauryl and stearyl,
examples of these alkyl-based groups are C₁₋₄ hydroxyalkyl groups such as 2-hydroxyethyl and 2-hydroxypropyl, and (C₁₋₄) alkoxy (C₁₋₄) alkyl groups such as methoxyethyl, ethoxyethyl and methoxypropyl,
• a C₃ to C₁₂ cycloalkyl group, such as an isobornyl group,
• a C₃ to C₂₀ aryl group such as a phenyl group,
• a C₄ to C₃₀ aralkyl group (C₁ to C₈ alkyl group) such as 2-phenylethyl or benzyl,
• a 4- to 12-membered heterocyclic group containing one or more hetero atoms chosen from O, N and S, the ring being aromatic or non-aromatic,
• a heterocyclylalkyl group (1 to 4 C alkyl), such as furfurylmethyl or tetrahydrofurfurylmethyl,
the said cycloalkyl, aryl, aralkyl, heterocyclic or heterocyclylalkyl groups possibly being optionally substituted with one or more substituents chosen from hydroxyl groups, halogen atoms and linear or branched alkyl groups of 1 to 4 C in which one or more hetero atoms chosen from O, N, S and P is(are) optionally intercalated, the said alkyl groups also possibly being optionally substituted with one or more substituents chosen from hydroxyl groups, halogen atoms (Cl, Br, I and F) and groups Si(R₄R₅), in which R₄ and R₅, which may be identical or different, represent a C₁ to C₆ alkyl group or a phenyl group,
examples of groups R₃ are methyl, ethyl, propyl, isobutyl, n-butyl, tert-butyl, isobutyl, hexyl, ethylhexyl, octyl, lauryl, isooctyl, isodecyl, dodecyl, cyclohexyl, t-butylcyclohexyl, t-butylbenzyl, isobornyl, phenyl, furfurylmethyl, tetrahydrofurfurylmethyl, 2-hydroxyethyl, 2-hydroxypropyl, 2-hydroxybutyl, methoxyethyl, ethoxyethyl, methoxyethyl, methoxypropyl and 2-ethyl-perfluorohexyl groups,
• another example of a group R₃ is the groups R₃ = -(OC₂H₄)ₘ-OR", with m = 5 to 150 and R" = H or C₁ to C₃₀ alkyl, for example -POE-methoxy; -POE-behenyl;
- the (meth)acrylamides of formula: 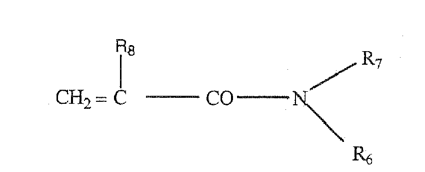 in which R₈ denotes H or methyl,
and R₇ and R₆, which may be identical or different, each represent a hydrogen atom or a linear or branched alkyl group of 1 to 18 carbon atoms, in which one or more hetero atoms chosen from 0, N, S and P is (are) optionally intercalated, the said alkyl group also possibly being optionally substituted with one or more substituents chosen from hydroxyl groups, halogen atoms (Cl, Br, I and F) and groups Si (R₄R₅) , in which R₄ and R₅, which may be identical or different, represent a C₁ to C₆ alkyl group or a phenyl group,
examples of these groups are methyl, ethyl, n-butyl, t-butyl, isopropyl, isohexyl, isooctyl, isononyl and C₁₋₄ hydroxyalkyl groups, such as 2-hydroxypropyl,
• a C₃ to C₁₂ cycloalkyl group, such as the isobornyl group,
• a C₃ to C₂₀ aryl group such as phenyl,
• a C₄ to C₃₀ aralkyl group (C₁ to C₈ alkyl group) such as 2-phenylethyl or benzyl,
• a 4- to 12-membered heterocyclic group containing one or more hetero atoms chosen from O, N and S, the ring being aromatic or non-aromatic,
• a heterocyclylalkyl group (1 to 4 C alkyl), such as furfurylmethyl or tetrahydrofurfurylmethyl,
the said cycloalkyl, aryl, aralkyl, heterocyclic or heterocyclylalkyl groups possibly being optionally substituted with one or more substituents chosen from hydroxyl groups, halogen atoms and linear or branched alkyl groups of 1 to 4 C in which one or more hetero atoms chosen from O, N, S and P is (are) optionally intercalated, the said alkyl groups also possibly being optionally substituted with one or more substituents chosen from hydroxyl groups, halogen atoms (Cl, Br, I and F) and groups Si (R₄R₅) , in which R₄ and R₅, which may be identical or different, represent a C₁ to C₆ alkyl group or a phenyl group,
examples of (meth)acrylamide monomers are (meth)acrylamide, N-ethyl(meth)acrylamide, N-butylacrylamide, N-t-butylacrylamide, N-isopropylacrylamide, N,N-dimethyl(meth)acrylamide, N,N-dibutylacrylamide, N-octylacrylamide, N-dodecylacrylamide, undecylacrylamide and N-(2-hydroxypropyl)methacrylamide;
- the allylic compounds of formula:
CH₂ = CH-CH₂-R₉ or CH₂ = C(CH₃)-CH₂-R₉;
- the vinyl compounds of formula:
CH₂ = CH-R₉,
in which R₉ is a group:
• hydroxyl,
• Cl,
• NH₂,
- OR₁₀, in which R₁₀ represents a phenyl group or a C₁ to C₁₂ alkyl group (the monomer is a vinyl ether or an allyl ether),
- acetamide: NHCOCH₃,
- OCOR₁₁, in which R₁₁ represents:
• a linear or branched alkyl group of 2 to 12 C (the monomer is a vinyl ester or an allylic ester),
• a C₃ to C₁₂ cycloalkyl group such as isobornyl or cyclohexyl,
• a C₃ to C₂₀ aryl group such as phenyl,
• a C₄ to C₃₀ aralkyl group (C₁ to C₈ alkyl group) such as 2-phenylethyl or benzyl,
• a 4- to 12-membered heterocyclic group containing one or more hetero atoms chosen from O, N and S, the ring being aromatic or non-aromatic,
• a heterocyclylalkyl group (1 to 4 C alkyl), such as furfurylmethyl or tetrahydrofurfurylmethyl, the said cycloalkyl, aryl, aralkyl, heterocyclic or heterocyclylalkyl groups possibly being optionally substituted with one or more substituents chosen from hydroxyl groups, halogen atoms and linear or branched alkyl groups of 1 to 4 C in which one or more hetero atoms chosen from 0, N, S and P is (are) optionally intercalated, the said alkyl groups also possibly being optionally substituted with one or more substituents chosen from hydroxyl groups, halogen atoms (Cl, Br, I and F) and groups Si (R₄R₅) , in which R₄ and R₅, which may be identical or different, represent a C₁ to C₆ alkyl group or a phenyl group,
examples of vinyl monomers are vinylcyclohexane and styrene,
examples of vinyl esters are: vinyl acetate, vinyl propionate, vinyl butyrate, vinyl ethylhexanoate, vinyl neononanoate and vinyl neododecanoate,
among the vinyl ethers are, for example, methyl vinyl ether, ethyl vinyl ether and isobutyl vinyl ether;
- (meth)acrylic or (meth) acrylamide or vinyl monomers containing a fluoro or perfluoro group, such as ethylperfluorooctyl methacrylate;
- silicone (meth)acrylic or vinyl monomers, such as methacryloxypropyltris(trimethylsiloxy)silane, acryloxypropylpolydimethylsiloxane or silicone (meth) acrylamides.

34. Copolymer according to Claim 33, in which the monomer(s) from which the block(s) B may be prepared is(are) chosen from the following monomers, for which the glass transition temperature of the corresponding homopolymer is in the range from -55°C to +55°C, such as :
hydroxyethyl methacrylate (Tg = 55°C), isobutyl methacrylate (Tg = 53°C), n-hexyl acrylate (Tg = 45°C), t-butyl acrylate (Tg = 50°C), vinyl acetate (Tg = 23°C), butyl methacrylate (Tg = 20°C), cyclohexyl acrylate (Tg = 19°C), hydroxyethyl acrylate (Tg = 15°C), methyl acrylate (Tg = 10°C), ethoxyethyl methacrylate (Tg = 0°C), n-hexyl methacrylate (Tg = -5°C), vinyl butyrate (Tg = -5°C), ethyl acrylate (Tg = -24°C), isobutyl acrylate (Tg = -24°C), methyl vinyl ether (Tg = -34°C), methoxyethyl acrylate (Tg = -33°C), n-hexyl acrylate (Tg = -45°C), n-butyl acrylate (Tg = -54°C), ethylhexyl acrylate (Tg = -50°C) and POE methacrylate (n=8 to 10) (Tg = -55°C); and from other monomers whose Tg is outside the range from -55 to +55°C, such as the following monomers: t-butylcyclohexyl acrylate, t-butylbenzyl acrylate, furfuryl acrylate and isobornyl acrylate; methyl methacrylate, ethyl methacrylate, cyclohexyl methacrylate, t-butylcyclohexyl methacrylate, t-butylbenzyl methacrylate and isobornyl methacrylate; N-butylacrylamide, N-t-butylacrylamide, N-isopropylacrylamide, N,N-dimethylacrylamide and N,N-dibutylacrylamide;
vinylcyclohexane, styrene;
hexyl acrylate, octyl acrylate, lauryl acrylate, isooctyl acrylate and isodecyl acrylate;
ethylhexyl methacrylate, octyl methacrylate, lauryl methacrylate, isooctyl methacrylate and isodecyl methacrylate;
vinyl neononanoate and vinyl neododecanoate;
N-octylacrylamide.

35. Copolymer according to any one of Claims 26 to 34, in which one or more of the blocks constituting the block(s) B is(are) chosen from hydrophilic monomers.

36. Copolymer according to any one of the preceding claims, in which one or more of the monomers B other than the block A is(are) hydrophilic.

37. Copolymer according to Claim 22 or Claim 35, in which the hydrophilic monomers are chosen from cationic monomers, anionic monomers and nonionic monomers.

38. Copolymer according to Claim 23, in which the non-hydrophilic monomer(s) of the block A is(are) chosen from the monomers constituting the block B, as defined in any one of Claims 25 to 33, while respecting the desired Tg and hydrophilicity criteria.

39. Copolymer according to Claim 37, in which the cationic monomers are chosen from 2-vinylpyridine; 4-vinylpyridine, dimethylaminoethyl (meth)acrylate; diethylaminoethyl (meth)acrylate; dimethylaminopropyl(meth)acrylamide; and salified or quaternized forms thereof.

40. Copolymer according to Claim 39, in which the anionic monomers are chosen from acrylic acid, methacrylic acid, crotonic acid, maleic anhydride, itaconic acid, fumaric acid, maleic acid, styrenesulfonic acid, 2-acrylamido-2-methylpropanesulfonic acid, vinylbenzoic acid, vinylphosphonic acid and sulfopropyl (meth)acrylate, and the salts thereof.

41. Copolymer according to Claim 39, in which the nonionic monomers are chosen from:
- ethylenic carboxybetaines or sulfobetaines obtained, for example, by quaternization of ethylenically unsaturated monomers comprising an amine function with carboxylic acid salts containing a labile halogen, for example sodium chloroacetate, or with cyclic sulfones, for example propane sulfone;
- hydroxyalkyl (meth)acrylates or hydroxyalkyl-(meth) acrylamides, the alkyl group of which contains from 2 to 4 C atoms, in particular hydroxyethyl (meth)acrylate or hydroxypropyl (meth)acrylate;
- (meth)acrylates or (meth)acrylamides of (C₁-C₄)-alkaxy(C₁₋₄)alkyl, such as methoxyethyl, ethoxyethyl and methoxypropyl,
- (meth)acrylates or (meth)acrylamides containing a group -(OC₂H₄)ₘ-OR", in which m = 5 to 150 and R" = H or C₁ to C₄ alkyl, for example -POE-methoxy; -POE-OH;
- vinyllactams, and
- polysaccharide meth(acrylates), for instance sucrose acrylate.

42. Copolymer according to any one of the preceding claims, chosen from diblock copolymers of AB type, triblock copolymers of ABA, BAB, ABC or ACB type, i.e. copolymers comprising, besides the blocks A and B, another block C other than the blocks A and B, multiblock copolymers containing more than three blocks of (AB)ₙ, (ABA)ₙ, (BAB)ₙ or (ABC)ₙ type, with C other than A or B, and multiblock copolymers containing more than three different blocks, of ABCD type.

43. Cosmetic composition comprising a linear block ethylenic copolymer comprising:
- at least one block A that may be prepared from monomers comprising from 52 to 100% by weight of an ethylenic monomer containing a lactam ring, corresponding to formula (I) below: 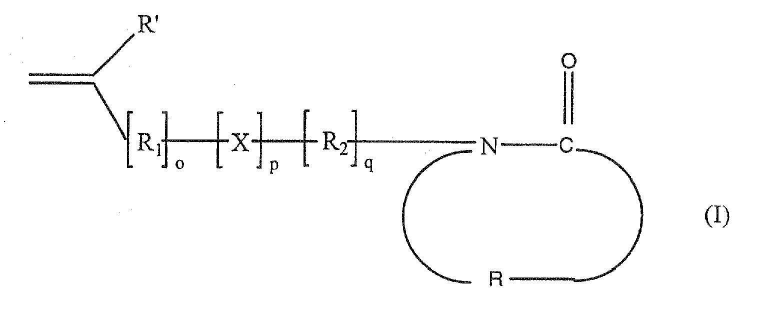 in which:
• R represents a group -(CH₂)ₙ-, in which one or more of the carbon atoms are optionally replaced with a nitrogen atom or any oxygen atom, in which n is an integer from 3 to 12, and in which one or more of the carbon atoms are optionally substituted with one or more C₁ to C₆ alkyl groups;
• R' represents H or a methyl group;
• R₁ and R₂, which may be identical or different, represent a linear, branched or cyclic alkylene or aralkylene group of 1 to 22 C, in which one or more of the carbon atoms are optionally replaced with
an oxygen or nitrogen atom;
• X is chosen from -OCO-, -NHCO-, -COO- and -O-;
• o, p and q represent, independently of each other, 0 or 1;
- and at least one block B that may be prepared from monomers not comprising an ethylenic monomer containing a lactam ring of formula (I), or comprising a minor proportion thereof, the said block B having a glass transition temperature Tg of between -55° and +55°, said block A representing 10 to 95% by weight of the copolymer and said block B representing 5 to 90% by weight of the copolymer.

44. Cosmetic composition according to claim 43, containing from 0.1% to 60% by weight, preferably from 0.5% to 50% by weight and more preferably from 1% to 40% by weight of the copolymer.

45. Composition according to either of claims 43 and 44, comprising, besides the said copolymer, a physiologically acceptable medium in which the copolymer is in dissolved or dispersed form.

46. Composition according to any one of claims 43 to 45, in which the physiologically acceptable medium comprises one or more suitable solvents forming a hydrophilic phase, chosen from water and mixtures of water and of hydrophilic organic solvent(s), such as alcohols and especially linear or branched lower monoalcohols containing from 2 to 5 carbon atoms, for instance ethanol, isopropanol or n-propanol, and polyols, for instance glycerol, diglycerol, propylene glycol, sorbitol, pentylene glycol and polyethylene glycols.

47. Composition according to claim 46, in which the hydrophilic phase also contains C₁ to C₂ ethers.

48. Cosmetic composition according to any one of claims 43 to 47, in which the said physiologically acceptable medium also comprises a fatty phase consisting of fatty substances that are liquid or solid at room temperature, of animal, plant, mineral or synthetic origin.

49. Composition according to any one of claims 43 to 48, also comprising one or more cosmetically acceptable organic solvents.

50. Cosmetic composition according to any one of claims 43 to 49, in which the said physiologically acceptable medium also comprises one or more auxiliary film-forming agents chosen from plasticizers and coalescers.

51. Cosmetic composition according to any one of claims 43 to 50, also comprising one or more dyestuffs chosen from water-soluble dyes and pulverulent dyestuffs, for instance pigments, nacres and flakes.

52. Composition according to any one of claims 43 to 51, also comprising fillers.

53. Cosmetic composition according to any one of claims 43 to 52, also comprising one or more ingredients commonly used in cosmetics, such as vitamins, thickeners, trace elements, softeners, sequestering agents, fragrances, acidifying or basifying agents, preserving agents, sunscreens, surfactants, antioxidants, agents for preventing hair loss, antidandruff agents, propellants, and film-forming or non-film-forming water-soluble or liposoluble polymers or polymers dispersed in water or in a fatty phase, or mixtures thereof.

54. Cosmetic composition according to any one of claims 43 to 53, **characterized in that** it is in the form of a suspension, a dispersion, a solution, a gel, an emulsion, especially an oil-in-water (O/W) or water-in-oil (W/O) emulsion, or a multiple emulsion (W/O/W or polyol/O/W or O/W/O emulsion), in the form of a cream, a paste, a mousse, a dispersion of vesicles, especially of ionic or nonionic lipids, a two-phase or multi-phase lotion, a spray, a powder, a paste, especially a soft paste or an anhydrous paste.

55. Cosmetic composition according to any one of claims 43 to 54, **characterized in that** it is a hair product, such as a lacquer or a shampoo.

56. Cosmetic composition according to any one of claims 43 to 55, **characterized in that** it is a makeup composition, such as a nail varnish.

57. Cosmetic process for making up or caring for keratin materials, comprising the application to the keratin materials of a composition according to one of claims 43 to 56.

58. Use of the copolymer according to claim 43 to improve the hairstyle hold of a hair lacquer, without tack.

59. Use of the copolymer according to claim 43 to improve the adhesion and the wear resistance of a nail varnish, without tack.

60. Use of the copolymer according to claim 43 in a cosmetic composition such as a makeup composition for masking wrinkles and giving the skin a smoothened appearance, without tautness.

## Patentansprüche

1. Lineares ethylenisches Sequenzcopolymer enthaltend:
- mindestens eine aus Monomeren herstellbare Sequenz A, die 52 bis 100 Gew.-% eines ethylenischen Monomers mit Lactamring der folgenden Formel (I) enthält: 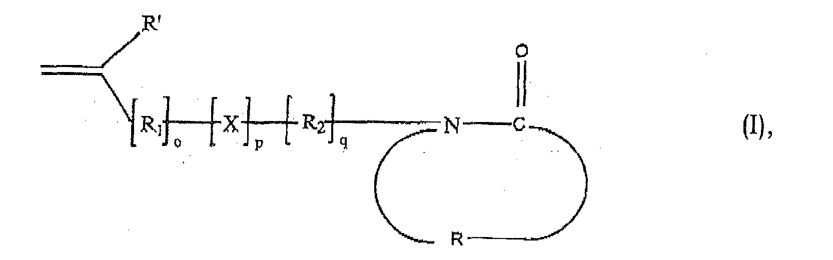 worin:
• R eine Gruppe -(CH₂)ₙ- bedeutet, wobei ein oder mehrere Kohlenstoffatome gegebenenfalls durch ein Stickstoffatom oder ein Sauerstoffatom ersetzt sein können, n eine ganze Zahl von 3 bis 12 bedeutet und ein oder mehrere Kohlenstoffatome gegebenenfalls mit einer oder mehreren C₁₋₆-Alkylgruppen substituiert sind;
• R' H oder die Methylgruppe bedeutet;
• R₁ und R₂, die gleich oder verschieden sein können, eine geradkettige, verzweigte oder cyclische Alkylen- oder Aralkylengruppe mit 1 bis 22 C bedeuten, worin ein oder mehrere Kohlenstoffatome gegebenenfalls durch ein Sauerstoffatom oder Stickstoffatom ersetzt sind;
• X unter -OCO-, -NHCO-, -COO- oder -O- ausgewählt ist; und
• o, p und q jeweils unabhängig voneinander 0 oder 1 bedeuten; und
- mindestens eine aus Monomeren herstellbare Sequenz B, die kein ethylenisches Monomer mit Lactamring der Formel (I) oder einen geringeren Anteil dieses Monomers enthält, wobei die Sequenz B eine Glasübergangstemperatur Tg von -55 bis +55 °C aufweist;
wobei die Sequenz A 10 bis 95 Gew.-% des Copolymers und die Sequenz B 5 bis 90 Gew.-% des Gesamtgewichts des Copolymers ausmacht.

2. Copolymer nach Anspruch 1, wobei in der Formel (I) o 0 bedeutet, p 1 ist, q 1 ist, R₂ -CH₂CH₂- bedeutet; X COO oder CONH bedeutet und R (CH₂)₃ oder (CH₂)₅ oder CH₂CH₂HN ist.

3. Copolymer nach Anspruch 1, wobei das ethylenische Monomer mit Lactamring ein Vinyllactam ist, das der folgenden Formel (II) entspricht: 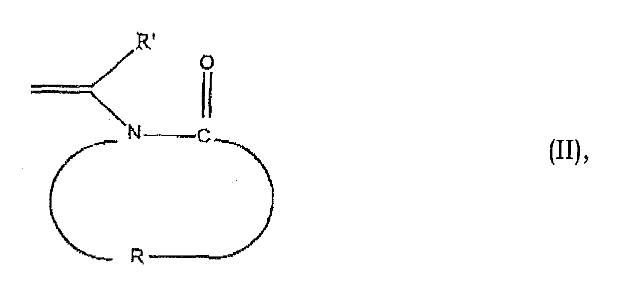 worin R und R' die in Anspruch 1 angegebenen Bedeutungen aufweisen.

4. Copolymer nach einem der Ansprüche 1 bis 4, wobei in den Formeln (I) und (II) die Gruppe R -(CH₂)ₙ-, wobei n eine ganze Zahl von 3 bis 5 ist, oder die Gruppe R -CH₂-CH₂-NH- bedeutet.

5. Copolymer nach einem der vorhergehenden Ansprüche, bei dem es sich um ein filmbildendes Copolymer handelt.

6. Copolymer nach einem der vorhergehenden Ansprüche, wobei das Copolymer insgesamt eine zahlenmittlere Molmasse von 4 000 bis 1 000 000, vorzugsweise 4 000 bis 800 000 und noch bevorzugter 4 000 bis 500 000 aufweist.

7. Copolymer nach einem der vorhergehenden Ansprüche, bei dem die Sequenz A 20 bis 90 Gew.-% des Gesamtgewichts des Copolymers ausmacht.

8. Copolymer nach einem der vorhergehenden Ansprüche, bei dem die Sequenz B 10 bis 80 Gew.-% des Gesamtgewichts des Copolymers ausmacht.

9. Copolymer nach einem der vorhergehenden Ansprüche, bei dem die zahlenmittlere Molmasse jeder Sequenz A oder B im Bereich von 2 000 bis 1 000 000, vorzugsweise 2 000 bis 800 000 und besser 2 000 bis 500 000 liegt.

10. Copolymer nach einem der vorhergehenden Ansprüche, bei dem die Monomere, aus denen die Sequenz A hergestellt wird, einen Anteil des ethylenischen Monomers mit Lactamring, beispielsweise des Vinyllactams der Formel (I) oder (II), von 50 Gew.-% oder darüber, bezogen auf das Gesamtgewicht der Monomere der Sequenz A, umfassen.

11. Copolymer nach Anspruch 8, bei dem der prozentuale Anteil des ethylenischen Monomers mit Lactamring, wie eines Vinyllactams, der Formel (I) oder (II) in der Sequenz A im Bereich von 55 bis 95 Gew.-% liegt und beispielsweise 90 Gew.-% beträgt.

12. Copolymer nach einem der vorhergehenden Ansprüche, bei dem die Sequenz A ein Homopolymer des ethylenischen Monomers mit Lactamring, beispielsweise eines N-Vinyllactams, der Formel (I) oder der Formel (II) ist.

13. Copolymer nach Anspruch 1, bei dem das N-Vinyllactam der Formel (I) das Pyrrilidinoethylacrylat oder Pyrrilidinoethylmethacrylat ist.

14. Copolymer nach Anspruch 2, bei dem das N-Vinyllactam der Formel (II) das N-Vinylpyrrolidon (n=3), N-Vinylpiperidinon (Valerolactam) (n=4), N-Vinylcaprolactam (n=5), N-Vinylimidazolidinon, worin R die Gruppe -CH₂-CH₂-NH- bedeutet, das N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, oder N-Vinyl-7-ethyl-2-caprolactam ist.

15. Copolymer nach Anspruch 14, wobei die Sequenz A ein N-Vinylpyrrolidon-Homopolymer ist.

16. Copolymer nach Anspruch 14, wobei die Sequenz A ein N-Vinylcaprolactam-Homopolymer ist.

17. Copolymer nach Anspruch 1, wobei die Sequenz A ein Copolymer ist, das aus Monomeren herstellbar ist und neben dem ethylenisch ungesättigten Monomer mit Lactamring, wie einem Vinyllactam, der Formel (I) ein oder mehrere weitere Monomere enthält.

18. Copolymer nach Anspruch 17, wobei die Sequenz A ein statistisches Copolymer, alternierendes Copolymer oder Gradientencopolymer ist.

19. Copolymer nach Anspruch 17 oder 18, wobei das oder die weiteren Monomere der Sequenz A unter den Monomeren ausgewählt sind, deren Glasübergangstemperatur des entsprechenden Homopolymers höchstens 50 °C, vorzugsweise höchstens 20 °C und besser höchstens 0 °C beträgt.

20. Copolymer nach Anspruch 19, wobei das oder die weiteren Monomere der Sequenz A ferner eine Glasübergangstemperatur des entsprechenden Homopolymers von mindestens -150 °C aufweisen.

21. Copolymer nach Anspruch 19, wobei das oder die weiteren Monomere des Sequenz A, bei denen die Glasübergangstemperatur des entsprechenden Homopolymers höchstens 50 °C beträgt, ausgewählt ist (sind) unter: *t*-Butylacrylat (Tg = 50 °C), Vinylacetat (Tg = 23 °C), Butylmethacrylat (Tg = 20 °C), Cyclohexylacrylat (19 °C), Hydroxyethylacrylat (15°C), Methylacrylat (Tg = 10 °C), Ethoxyethylmethacrylat (Tg = 0 °C), n-Hexylmethacrylat (-5°C), Ethylacrylat (Tg = -24 °C), Isobutylacrylat (-24 °C), Vinylmethylether (-34 °C), Methoxyethylacrylat (-33 °C), *n*-Butylacrylat (Tg = -54 °C), Ethylhexylacrylat (Tg = -50 °C), PEO-Methacrylat (n = 8 bis 10) (Tg = -55 °C) und Isobornylmethacrylat; Butylacrylamid, N-Isopropylacrylamid, N,N-Dimethylacrylamid und N,N-Dibutylacrylamid, Hexylacrylat, Octylacrylat, Laurylacrylat, Isooctylacrylat, Ethylhexylmethacrylat, Octylmethacrylat, Laurylmethacrylat, Vinylneononanoat, Vinylneododecanoat; und N-Octylacrylamid.

22. Copolymer nach Anspruch 17, wobei ein oder mehrere der weiteren Monomere der Sequenz A unter den hydrophilen Monomeren ausgewählt sind.

23. Copolymer nach Anspruch 17, wobei ein oder mehrere der weiteren Monomere der Sequenz A unter den nicht hydrophilen Monomeren ausgewählt sind.

24. Copolymer nach einem der vorhergehenden Ansprüche, bei dem die Sequenz A eine Glasübergangstemperatur der Sequenz insgesamt von 0 bis 250 °C, vorzugsweise 0 bis 220 °C und noch bevorzugter 5 bis 200 °C aufweist.

25. Copolymer nach einem der vorhergehenden Ansprüche, wobei die Sequenz A eine hydrophile Sequenz ist.

26. Copolymer nach einem der vorhergehenden Ansprüche, wobei die von der Sequenz A verschiedene(n) Sequenz(en) B aus einem oder mehreren ethylenisch ungesättigten Monomeren herstellbar ist (sind), die ausgewählt sind unter: Allylmonomeren, Acrylaten, Methacrylaten, Acrylamiden, Methacrylamiden, Vinylmonomeren und deren Gemischen und gegebenenfalls ethylenisch ungesättigten Monomeren mit Lactamring, beispielsweise Vinyllactammonomeren, der Formel (I) oder (II), wobei der Gewichtsanteil der Monomere (I) oder (II) unter 50 Gew.-% liegt und vorzugsweise höchstens 40 Gew.-% und noch bevorzugter höchstens 30 Gew.-% beträgt.

27. Copolymer nach einem der vorhergehenden Ansprüche, wobei die Sequenz B eine Glasübergangstemperatur Tg aufweist, die im Bereich von -50 bis +50 °C und vorzugsweise -50 bis +45 °C liegen kann.

28. Copolymer nach einem der vorhergehenden Ansprüche, wobei das oder die Monomer(e), die die Sequenz B bilden, eine Länge der Kohlenstoffkette von höchsten 10 Kohlenstoffatomen aufweisen.

29. Copolymer nach einem der vorhergehenden Ansprüche, bei dem das oder die Monomer(e), die die Sequenz B bilden, eine Länge der Kohlenstoffkette von mindestens 12 Kohlenstoffatomen aufweisen und der Anteil dieser Monomere in der Sequenz B unter 50 Gew.-% liegt.

30. Copolymer nach einem der vorhergehenden Ansprüche, worin die Sequenz B mit einer Glasübergangstemperatur Tg von -55 bis +55 °C, bei der es sich um ein Homopolymer oder ein Copolymer handelt, von einem oder mehreren Monomeren abgeleitet ist, die so gewählt sind, dass die aus diesen Monomeren hergestellten Homopolymere Glasübergangstemperaturen von -200 bis +200 °C aufweisen können.

31. Copolymer nach Anspruch 30, wobei die Sequenz B aus einem Copolymer gebildet ist, das aus einem ersten Monomer, dessen Tg des entsprechenden Homopolymers im Bereich von +20 bis +250 °C liegt, beispielsweise Methylmethacrylat (MMA) oder Styrol, und einem zweiten Monomer aufgebaut wird, dessen Tg des entsprechenden Homopolymers im Bereich von -200 bis +20 °C liegt.

32. Copolymer nach Anspruch 30, wobei die Sequenz B, deren Glasübergangstemperatur im Bereich von -55 bis +55 °C liegt, ein Homopolymer ist, das aus nur einem Monomertyp aufgebaut wird, wobei die Tg des entsprechenden Homopolymers im Bereich von -55 bis +55 °C, vorzugsweise -50 bis +50 °C und vorzugsweise -50 bis +45 °C liegt.

33. Copolymer nach einem der vorhergehenden Ansprüche, bei dem das oder die Monomere, aus dem oder denen die Sequenz(en) B herstellbar ist (sind), unter den folgenden Monomeren ausgewählt ist (sind):
- ethylenisch ungesättigten Kohlenwasserstoffen mit 2 bis 10 C, wie Ethylen, Isopren oder Butadien;
- Acrylaten der Formel CH₂=CHCOOR₃;
- Methacrylaten der Formel: 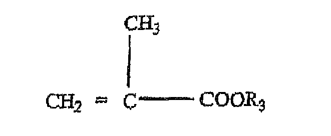 worin R₃ bedeutet:
• eine geradkettige oder verzweigte Alkylgruppe mit 1 bis 18 Kohlenstoffatomen, in die gegebenenfalls ein oder mehrere Heteroatome, die unter O, N, S und P ausgewählt sind, eingebaut sind,
wobei die Alkylgruppe gegebenenfalls ferner mit einem oder mehreren Substituenten substituiert sein kann, die unter Hydroxy, Halogenatomen (Cl, Br, I und F) und Si(R₄R₅) ausgewählt sind, wobei R₄ und R₅, die gleich oder verschieden sind, eine C₁₋₆-Alkylgruppe oder eine Phenylgruppe bedeuten, wobei Beispiele für solche Alkylgruppen Methyl, Ethyl, Propyl, Butyl, Isobutyl, *t*-Butyl, Ethylhexyl, Octyl, Lauryl und Stearyl sind, und
Beispiele für von Alkylgruppen abgeleitete Gruppen C₁₋₄-Hydroxyalkylgruppen, wie 2-Hydroxyethyl und 2-Hydroxypropyl, und Alkoxy(C₁₋₄)alkyl(C₁₋₄)gruppen, wie Methoxyethyl, Ethoxyethyl und Methoxypropyl, sind
• eine Cycloalkylgruppe mit 3 bis 12 Kohlenstoffatomen, wie Isobornyl,
• eine Arylgruppe mit 3 bis 20 Kohlenstoffatomen, wie Phenyl,
• eine Aralkylgruppe mit 4 bis 30 Kohlenstoffatomen (C₁₋₈-Alkylgruppe), beispielsweise 2-Phenylethyl oder Benzyl,
• eine heterocyclische Gruppe mit 4 bis 12 Bestandteilen, die ein oder mehrere Heteroatome enthält, die unter O, N und S ausgewählt sind, wobei der Ring aromatisch oder nichtaromatisch sein kann,
• eine Heterocyclylalkylgruppe (Alkyl mit 1 bis 4 C), wie Furfurylmethyl oder Tetrahydrofurfurylmethyl,
wobei die Cycloalkylgruppen, Arylgruppen, Aralkylgruppen, heterocyclischen Gruppen oder Heterocyclylalkylgruppen gegebenenfalls mit einem oder mehreren Substituenten substituiert sein können, die unter Hydroxy, Halogenatomen und geradkettigen oder verzweigten C₁₋₄-Alkylgruppen ausgewählt sind, in die gegebenenfalls ein oder mehrere Heteroatome, die unter O, N, S und P ausgewählt sind, eingebaut sein können, wobei die Alkylgruppen ferner gegebenenfalls mit einem oder mehreren Substituenten substituiert sein können, die unter Hydroxy, Halogenatomen (C1, Br, I und F) und Si(R₄R₅) ausgewählt sind, wobei R₄ und R₅, die gleich oder verschieden sind, C₁₋₆-Alkyl oder Phenyl bedeuten,
wobei Beispiele für Gruppen R₃ sind: Methyl, Ethyl, Propyl, Isobutyl, n-Butyl, *t*-Butyl, Isobutyl, Hexyl, Ethylhexyl, Octyl, Lauryl, Isooctyl, Isodecyl, Dodecyl, Cyclohexyl, t-Butylcyclohexyl, *t*-Butylbenzyl, Isobornyl, Phenyl, Furfurylmethyl, Tetrahydrofurfurylmethyl, 2-Hydroxyethyl, 2-Hydroxypropyl, 2-Hydroxybutyl, Methoxyethyl, Ethoxyethyl, Methoxyethyl, Methoxypropyl, 2-Ethylperfluorhexyl,
• ein weiteres Beispiel für die Gruppe R₃ sind Gruppen R₃ = -(OC₂H₄)ₘ-OR" mit m = 5 bis 150 und R" = H oder C₁-₃₀-Alkyl, beispielsweise -PEO-Methoxy; -PEO-Behenyl;
- (Meth)acrylamiden der Formel: 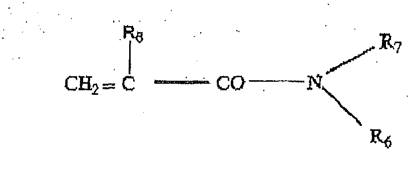 worin
R₈ H oder Methyl bedeutet;
und R₇ und R₆, die gleich oder verschieden sind, jeweils ein Wasserstoffatom oder eine geradkettige oder verzweigte Alkylgruppe mit 1 bis 18 Kohlenstoffatomen bedeuten, in die gegebenenfalls ein oder mehrere Heteroatome, die unter O, N, S und P ausgewählt sind, eingebaut sind, und wobei die Alkylgruppe ferner gegebenenfalls mit einem oder mehreren Substituenten substituiert sein kann, die unter Hydroxygruppen, Halogenatomen (Cl, Br, I und F) und Gruppen Si(R₄R₅) ausgewählt sind, wobei R₄ und R₅, die gleich oder verschieden sind, C₁₋₆-Alkyl oder Phenyl bedeuten,
wobei Beispiele für diese Gruppen Methyl, Ethyl, n-Butyl, *t*-Butyl, Isopropyl, Isohexyl, Isooctyl, Isononyl und C₃₋₄-Hydroxyalkyl, wie 2-Hydroxypropyl, sind,
• Cycloalkyl mit 3 bis 12 Kohlenstoffatomen, wie Isobornyl,
• C₃₋₂₀-Aryl, wie Phenyl,
• Aralkyl mit 4 bis 30 Kohlenstoffatomen (Alkyl mit 1 bis 8 Kohlenstoffatomen), wie 2-Phenylethyl oder Benzyl,
• eine heterocyclische Gruppe mit 4 bis 12 Bestandteilen, die ein oder mehrere Heteroatome enthält, die unter O, N und S ausgewählt sind, wobei der Ring aromatisch oder nichtaromatisch ist,
• eine Heterocyclylalkylgruppe (Alkyl mit 1 bis 4 C), wie beispielsweise Furfurylmethyl oder Tetrahydrofurfurylmethyl,
wobei die Cycloalkylgruppen, Arylgruppen, Aralkylgruppen, heterocyclischen Gruppen oder Heterocyclylalkylgruppen gegebenenfalls mit einem oder mehreren Substituenten substituiert sein können, der (die) unter den Hydroxygruppen, Halogenatomen und geradkettigen oder verzweigten Alkylgruppen mit 1 bis 4 C ausgewählt sind, in die gegebenenfalls ein oder mehrere Heteroatome, die unter O, N, S und P ausgewählt sind, eingebaut sind, wobei die Alkylgruppen ferner gegebenenfalls mit einem oder mehreren Substituenten substituiert sind, die unter den Hydroxygruppen, Halogenatomen (Cl, Br, I und F) und den Gruppen Si(R₄R₅) ausgewählt sind, wobei R₄ und R₅, die gleich oder verschieden sind, eine C₁₋₆-Alkylgruppe oder Phenyl bedeuten,
wobei Beispiele für (Meth)acrylamidmonomere (Meth)acrylamid, N-Ethyl(meth)acrylamid, N-Ethyl(meth)acrylamid, N-Butylacrylamid, N-*t*-butylacrylamid, N-Isopropylacrylamid, N,N-Dimethyl(meth)acrylamid, N,N-Dibutylacrylamid, N-Octylacrylamid, N-Dodecylacrylamid, Undecylacrylamid und N-(2-Hydroxypropylmethacrylamid) sind;
- Allylverbindungen der Formel: CH₂ = CH-CH₂-R₉ oder CH₂ = C(CH₃)-CH₂-R₉;
- Vinylverbindungen der Formel: CH₂ = CH-R₉, worin R₉ bedeutet:
- Hydroxy,
- Cl.
- NH₂,
- OR₁₀, wobei Rio eine Phenylgruppe oder eine C₁₋₁₂-Alkylgruppe ist (das Monomer ist ein Vinylether oder Allylether),
- Acetamid: NHCOCH₃,
- OCOR₁₁, wobei R₁₁ bedeutet:
• eine geradkettige oder verzweigte Alkylgruppe mit 2 bis 12 C (das Monomer ist ein Vinylester oder Allylester);
• eine Cycloalkylgruppe mit 3 bis 12 Kohlenstoffatomen, wie Isobornyl, Cyclohexyl,
• eine Arylgruppe mit 3 bis 20 Kohlenstoffatomen, wie Phenyl,
• eine Aralkylgruppe mit 4 bis 30 Kohlenstoffatomen (Alkylgruppe mit 1 bis 8 C), wie 2-Phenylethyl; Benzyl,
• eine heterocyclische Gruppe mit 4 bis 12 Bestandteilen, die ein oder mehrere Heteroatome enthält, die unter O, N und S ausgewählt sind,
wobei der Ring aromatisch oder nichtaromatisch sein kann,
• eine Heterocyclylalkylgruppe (Alkyl mit 1 bis 4 C), wie beispielsweise Furfurylmethyl oder Tetrahydrofurfurylmethyl,
wobei die Cyclalkylgruppen, Arylgruppen, Aralkylgruppen, heterocyclischen Gruppen oder Heterocyclylalkylgruppen gegebenenfalls mit einem oder mehreren Substituenten substituiert sein können, der unter den Hydroxygruppen, Halogenatomen und geradkettigen oder verzweigten Alkylgruppen mit 1 bis 4 C ausgewählt sind, in die gegebenenfalls ein oder mehrere Heteroatome eingebaut sind, die unter O, N, S und P ausgewählt sind, wobei die Alkylgruppen ferner gegebenenfalls mit einem oder mehreren Substituenten substituiert sein können, die unter Hydroxygruppen, Halogenatomen (C1, Br, I und F) und Gruppen Si(R₄R₅) ausgewählt sind, wobei R₄ und R₅, die gleich oder verschieden sind, C₁₋₆-Alkyl oder Phenyl bedeuten,
wobei Beispiele für Vinylmonomere Vinylcyclohexan und Styrol sind,
Beispiele für Vinylester sind: Vinylacetat, Vinylpropionat, Vinylbutyrat, Vinylethylhexanoat, Vinylneononanoat; und Vinylneododecanoat,
von den Vinylestern sind beispielsweise Vinylmethylether, Vinylethylether und Vinylisobutylether zu nennen;
- (Meth)acrylmonomeren oder (Meth)acrylamiden oder Vinylmonomeren mit fluorierter oder perfluorierter Gruppe, beispielsweise Ethylperfluoroctylmethacrylat;
- (Meth)acrylamonomeren oder Vinylmonomeren, die siliconiert sind, beispielsweise Methacryloxypropyltris(trimethylsiloxy)-silan, Acryloxypropylpolydimethylsiloxan oder (Meth)acrylamiden, die siliconiert sind.

34. Copolymer nach Anspruch 33, wobei das oder die Monomer(e), ausgehend von dem oder denen die Sequenz(en) B herstellbar ist (sind), unter den folgenden Monomeren ausgewählt ist (sind), deren Glasübergangstemperatur des entsprechenden Homopolymers im Bereich von -55 bis +55 °C liegt, wie: Hydroxyethylmethacrylat (55 °C), Isobutylmethacrylat (53°C), N-Hexylacrylat (Tg = 45 °C), t-Butylacrylat (Tg = 50 °C), Vinylacetat (Tg = 23 °C), Butylmethacrylat (Tg = 20 °C), Cyclohexylacrylat (19 °C), Hydroxyethylacrylat (15 °C), Methylacrylat (Tg = 10 °C), Ethoxyethylmethacrylat (Tg = 0 °C), n-Hexylmethacrylat (-5 °C), Vinylbutyrat (-5 °C), Ethylacrylat (Tg = -24 °C), Isobutylacrylat (-24 °C), Vinylmethylether (-34 °C), Methoxyethylacrylat (-33 °C), n-Hexylacrylat (-45 °C), n-Butylacrylat (Tg = -54 °C), Ethylhexylacrylat (Tg = -50 °C), PEO-methacrylat (n = 8 bis 10) (Tg =
- 55 °C); und weiteren Monomeren, deren Tg außerhalb des Bereichs von -55 bis +55 °C liegt, beispielsweise den folgenden Monomeren: *t*-Butylcyclohexylacrylat, *t*-Butylbenzylacrylat, Furfurylacrylat und Isobornylacrylat; Methylmethacrylat, Ethylmethacrylat, Cyclohexylmethacrylat, *t*-Butylcyclohexylmethacrylat, *t*-Butylbenzylmethacrylat und Isobornylmethacrylat; N-Butylacrylamid, N-*t*-Butylacrylamid, N-Isopropylacrylamid, N,N-Dimethylacrylamid und N,N-Dibutylacrylamid; Vinylcyclohexan, Styrol; Hexylacrylat, Octylacrylat, Laurylacrylat, Isooctylacrylat, Isodecylacrylat; Ethylhexylmethacrylat, Octylmethacrylat, Laurylmethacrylat, Isooctylmethacrylat, Isodecylmethacrylat; Vinylneononanoat, Vinylneododecanoat; N-Octylacrylamid.

35. Copolymer nach einem der Ansprüche 26 bis 34, wobei ein oder mehrere der Monomere, die die Sequenz(en) B aufbauen, unter den hydrophilen Monomeren ausgewählt sind.

36. Copolymer nach einem der vorhergehenden Ansprüche, wobei ein oder mehrere der Sequenzen B, die von der Sequenz A verschieden sind, hydrophil sind.

37. Copolymer nach Anspruch 22 oder nach Anspruch 35, bei dem die hydrophilen Monomere unter den kationischen Monomeren, anionischen Monomeren und nichtionischen Monomeren ausgewählt sind.

38. Copolymer nach Anspruch 23, bei dem das oder die nicht hydrophilen Monomere der Sequenz A unter den in den Ansprüchen 25 bis 33 definierten Monomeren ausgewählt sind, wobei die Kriterien hinsichtlich der Tg und der Hydrophilie, die gewünscht sind, beachtet werden.

39. Copolymer nach Anspruch 37, wobei die kationischen Monomere unter 2-Vinylpyridin; 4-Vinylpyridin, Dimethylaminoethyl(meth)-acrylat; Diethylaminoethyl(meth)acrylat; Dimethylaminopropyl(meth)acrylamid und den in Form der Salze vorliegenden Formen oder quaternisierten Formen dieser Monomere ausgewählt sind.

40. Copolymer nach Anspruch 39, wobei die anionischen Monomere unter Acrylsäure, Methacrylsäure, Crotonsäure, Maleinsäureanhydrid, Itaconsäure, Fumarsäure, Maleinsäure, Styrolsulfonsäure, 2-Acrylamido-2-methylpropansulfonsäure, Vinylbenzoesäure, Vinylphosphonsäure, Sulfopropyl(meth)acrylat und den Salzen dieser Monomere ausgewählt sind.

41. Copolymer nach Anspruch 39, wobei die nichtionischen Monomere ausgewählt sind unter:
- ethylenisch ungesättigten Carboxybetainen oder Sulfobetainen, die beispielsweise durch Quaternisierung von Monomeren mit ethylenisch ungesättigter Bindung, die eine Aminfunktion enthalten, mit Carbonsäuresalzen mit mobilem Halogen, beispielsweise Natriumchloracetat, oder mit cyclischen Sulfonen, beispielsweise Propansulfon;
- Hydroxyalkyl(meth)acrylaten oder Hydroxyalkyl(meth)acrylamiden, bei denen die Alkylgruppe 2 bis 4 C-Atome aufweist, insbesondere Hydroxymethyl(meth)acrylat oder Hydroxypropyl(meth)acrylat,
- Alkoxy(C₁₋₄)alkyl(C₁₋₄)(meth)acrylate oder -(meth)acrylamide, beispielsweise Methoxyethyl(meth)acrylat oder -(meth)acrylamid, Ethoxyethyl(meth)acrylat oder -(meth)acrylamid oder Methoxypropyl(meth)acrylat oder -(meth)acrylamid,
- (Meth)acrylate oder (Meth)acrylamide mit einer Gruppe
- (OC₂H₄)ₘ-OR" mit 5 = 5 bis 150 und R" = H oder C₁₋₄-Alkyl, beispielsweise -PEO-Methoxy, PEO-OH;
- Vinyllactamen; und
- (Meth)acrylaten von Polysacchariden, wie Saccharoseacrylat.

42. Copolymer nach einem der vorhergehenden Ansprüche, das unter den bisequentiellen Copolymeren vom Typ AB, trisequentiellen Copolymeren vom Typ ABA, BAB, ABC, ACB, d. h. Copolymeren, die neben den Sequenzen A und B eine von den Sequenzen A und B verschiedene Sequenz C aufweisen, multisequentiellen Copolymeren mit mehr als 3 Sequenzen vom Typ (AB)ₙ, (ABA)ₙ, (BAB)ₙ, (ABC)ₙ, wobei C von A oder B verschieden ist, und multisequentiellen Copolymeren ausgewählt ist, die mehr als drei unterschiedliche Sequenzen vom Typ ABCD aufweisen.

43. Kosmetische Zusammensetzung, die ein lineares ethylenisches Sequenzcopolymer enthält, das aufweist:
- mindestens eine aus Monomeren herstellbare Sequenz A, die 52 bis 100 Gew.-% eines ethylenischen Monomers mit Lactamring der folgenden Formel (I) enthält: 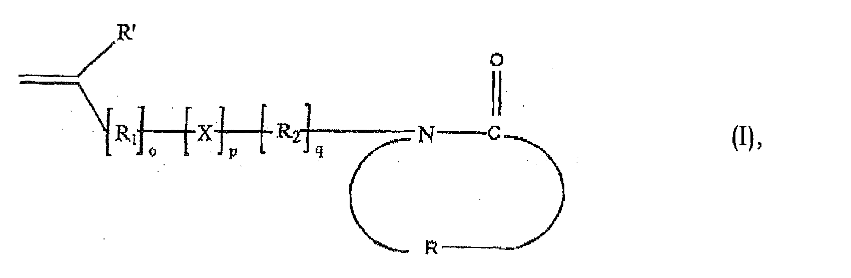 worin:
• R eine Gruppe -(CH₂)ₙ- bedeutet, wobei ein oder mehrere Kohlenstoffatome gegebenenfalls durch ein Stickstoffatom oder ein Sauerstoffatom ersetzt sein können, n eine ganze Zahl von 3 bis 12 bedeutet und ein oder mehrere Kohlenstoffatome gegebenenfalls mit einer oder mehreren C₁₋₆-Alkylgruppen substituiert sind;
• R' H oder die Methylgruppe bedeutet;
• R₁ und R₂, die gleich oder verschieden sind, eine geradkettige, verzweigte oder cyclische Alkylen- oder Aralkylengruppe mit 1 bis 22 C bedeuten, worin ein oder mehrere Kohlenstoffatome gegebenenfalls durch ein Sauerstoffatom oder Stickstoffatom ersetzt sind;
• X unter -OCO-, -NHCO-, -COO- oder -O- ausgewählt ist; und
• o, p und q jeweils unabhängig voneinander 0 oder 1 bedeuten; und
- mindestens eine aus Monomeren herstellbare Sequenz B, die kein ethylenisches Monomer mit Lactamring der Formel (I) oder einen geringeren Anteil dieses Monomers enthält, wobei die Sequenz B eine Glasübergangstemperatur Tg von -55 bis +55 °C aufweist; wobei die Sequenz A und die Sequenz B 1 bis 99 Gew.-% des Gesamtgewichts des Copolymers ausmachen.

44. Kosmetische Zusammensetzung nach Anspruch 43, die 0,1 bis 60 Gew.-%, vorzugsweise 0,5 bis 50 Gew.-% und noch bevorzugter 1 bis 40 Gew.-% des Copolymers enthält.

45. Zusammensetzung nach einem der Ansprüche 43 und 44, die neben dem Copolymer ein physiologisch akzeptables Medium aufweist, in dem das Copolymer in gelöster oder dispergierter Form vorliegt.

46. Zusammensetzung nach einem der Ansprüche 43 bis 45, wobei das physiologisch akzeptable Medium ein oder mehrere geeignete Lösungsmittel enthält, die eine hydrophile Phase bilden und die unter Wasser und Gemischen von Wasser und einem oder mehreren hydrophilen organischen Lösungsmitteln ausgewählt sind, wie Alkoholen und insbesondere geradkettigen oder verzweigten niederen Monoalkoholen mit 2 bis 5 Kohlenstoffatomen, beispielsweise Ethanol, Isopropanol oder *n*-Propanol, und Polyolen, wie Glycerin, Diglycerin, Propylenglycol, Sorbit, Pentylenglycol und Polyethylenglycolen.

47. Zusammensetzung nach Anspruch 46, wobei die hydrophile Phase ferner C₁₋₂-Ether enthält.

48. Kosmetische Zusammensetzung nach einem der Ansprüche 43 bis 47, wobei das physiologisch akzeptable Medium ferner eine Fettphase umfasst, die aus bei Umgebungstemperatur flüssigen oder festen Fettsubstanzen tierischer, pflanzlicher, mineralischer oder synthetischer Herkunft zusammengesetzt ist.

49. Zusammensetzung nach einem der Ansprüche 43 bis 48, die ferner ein oder mehrere kosmetisch akzeptable organische Lösungsmittel enthält.

50. Kosmetische Zusammensetzung nach einem der Ansprüche 43 bis 49, wobei das physiologisch akzeptable Medium ferner ein oder mehrere Hilfsstoffe für die Filmbildung aufweist, die unter den Weichmachern und Koaleszenzmitteln ausgewählt sind.

51. Kosmetische Zusammensetzung nach einem der Ansprüche 43 bis 50, die ferner ein oder mehrere Farbmittel aufweist, die unter den wasserlöslichen Farbstoffen und pulverförmigen Farbstoffen, wie Pigmenten, Perlglanzstoffen und Pailletten ausgewählt sind.

52. Zusammensetzung nach einem der Ansprüche 43 bis 51, die ferner Füllstoffe enthält.

53. Kosmetische Zusammensetzung nach einem der Ansprüche 43 bis 52, die ferner einen oder mehrere Bestandteile enthält, die häufig in der Kosmetik verwendet werden, wie Vitamine, Verdickungsmittel, Spurenelemente, beruhigende Stoffe, Maskierungsmittel, Parfums, Alkalisierungsmittel, Ansäuerungsmittel, Konservierungsmittel, Sonnenschutzfilter, grenzflächenaktive Stoffe, Antioxidantien, Wirkstoffe gegen Haarausfall, Antischuppenmittel, Treibmittel, wasserlösliche Polymere, fettlösliche Polymere oder Polymere in Dispersion in Wasser oder in einer Fettphase, die filmbildend oder nicht filmbildend sind, oder deren Gemische.

54. Kosmetische Zusammensetzung nach einem der Ansprüche 43 bis 53, **dadurch gekennzeichnet, dass** sie als Suspension, Dispersion, Lösung, Gel, Emulsion, insbesondere Öl-in-Wasser-Emulsion (O/W) oder Wasser-in-Öl-Emulsion (W/O) oder multiple Emulsion (W/O/W oder Polyol/O/W oder O/W/O), als Creme, Paste, Schaum, Vesikeldispersion insbesondere von ionischen oder nichtionischen Lipiden, zweiphasige oder mehrphasige Lotion, Spray, Pulver, Paste und insbesondere weiche Paste oder wasserfreie Paste vorliegt.

55. Kosmetische Zusammensetzung nach einem der Ansprüche 43 bis 54, **dadurch gekennzeichnet, dass** es sich um ein Produkt für die Haarbehandlung, beispielsweise einen Haarlack oder ein Haarwaschmittel handelt.

56. Kosmetische Zusammensetzung nach einem der Ansprüche 43 bis 55, **dadurch gekennzeichnet, dass** es sich um eine Zusammensetzung zum Schminken handelt, beispielsweise einen Nagellack.

57. Kosmetisches Verfahren zum Schminken oder für die Pflege von Keratinsubstanzen, das das Aufbringen einer Zusammensetzung nach einem der Ansprüche 43 bis 56 auf die Keratinsubstanzen umfasst.

58. Verwendung des Copolymers, wie es in Anspruch 43 definiert wurde, um, ohne zu kleben, die Festigungswirkung eines Haarlacks zu verbessern.

59. Verwendung des Copolymers, wie es in Anspruch 43 definiert wurde, um die Haftung und Abriebfestigkeit eines Nagellacks zu verbessern, ohne dass dieser klebt.

60. Verwendung des Copolymers, wie es in Anspruch 43 definiert wurde, in einer kosmetischen Zusammensetzung, beispielsweise einer Zusammensetzung zum Schminken, um Falten zu kaschieren und der Haut, ohne zu spannen, ein glattes Aussehen zu geben.
